(19) Europäisches Patentamt / European Patent Office / Office européen des brevets

(11) **EP 3 975 733 B1**

(12) **EUROPEAN PATENT SPECIFICATION**

(45) Date of publication and mention
of the grant of the patent:
**09.08.2023 Bulletin 2023/32**

(21) Application number: **20733349.3**

(22) Date of filing: **29.05.2020**

(51) International Patent Classification (IPC):
**A23J 3/34** (2006.01)     **A23L 2/66** (2006.01)
**A23L 33/18** (2016.01)

(52) Cooperative Patent Classification (CPC):
**A23J 3/343; A23L 2/66; A23L 33/18**

(86) International application number:
**PCT/EP2020/065022**

(87) International publication number:
**WO 2020/239998 (03.12.2020 Gazette 2020/49)**

(54) **PALATABLE EXTENSIVELY HYDROLYSED WHEY PROTEIN HYDROLYSATES**

WOHLSCHMECKENDE, BETRÄCHTLICH HYDROLYSIERTE MOLKEPROTEINHYDROLYSATE

HYDROLYSATS DE PROTÉINES DE LACTOSÉRUM FORTEMENT HYDROLYSÉS AU GOÛT AGRÉABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.05.2019 EP 19177371**
**07.02.2020 EP 20156159**

(43) Date of publication of application:
**06.04.2022 Bulletin 2022/14**

(60) Divisional application:
**23183496.1**

(73) Proprietor: **Arla Foods amba**
**8260 Viby J (DK)**

(72) Inventors:
• **SØRENSEN, Hans Peter**
**6920 Videbæk (DK)**
• **BERTELSEN, Hans**
**6920 Videbæk (DK)**
• **SØRENSEN, Anetta Kynde**
**6920 Videbæk (DK)**

(74) Representative: **Plougmann Vingtoft a/s**
**Strandvejen 70**
**2900 Hellerup (DK)**

(56) References cited:
WO-A1-93/24020          WO-A1-2016/156077
WO-A1-2018/164254       RU-C2- 2 529 707
US-A1- 2004 047 947

• DATABASE GNPD [Online] MINTEL; 11 January 2019 (2019-01-11), anonymous: "Lightly Sparkling Cranberry Flavoured Drink", XP055730002, retrieved from www.gnpd.com Database accession no. 6259085
• DHAVAL ANUSHA ET AL: "Potential Applications of Food Derived Bioactive Peptides in Management of Health", INTERNATIONAL JOURNAL OF PEPTIDE RESEARCH AND THERAPEUTICS, SPRINGER-VERLAG, DORDRECHT, NL, vol. 22, no. 3, 19 February 2016 (2016-02-19), pages 377-398, XP036022224, ISSN: 1573-3149, DOI: 10.1007/S10989-016-9514-Z [retrieved on 2016-02-19]
• DATABASE GNPD [Online] MINTEL; 1 April 2019 (2019-04-01), anonymous: "Soda Flavour Carbonated Soft Drink", XP055730005, Database accession no. 6439361
• DATABASE GNPD [Online] MINTEL; 11 January 2019 (2019-01-11), anonymous: "Lightly Sparkling Cranberry Flavoured Drink", XP055730002, Database accession no. 6259085

**Description**

**Technical field of the invention**

The present invention relates to new whey protein hydrolysates that have a high degree of hydrolysis, are palatable and have a low turbidity without being subjected to ultrafiltration. The invention furthermore relates to methods of preparing the new whey protein hydrolysates, uses of the new whey protein hydrolysates and a carbonated beverage comprising these new whey protein hydrolysates.

**Background of the invention**

It is well known to use whey protein hydrolysates as ingredients in various food products. Whey protein hydrolysates are commonly prepared by hydrolysing a whey protein substance, such as a whey protein isolate or whey protein concentrate, with a food grade proteolytic and/or peptidolytic preparation to a desired degree of hydrolysis. In some situations, it is desired to prepare a whey protein hydrolysate with a high degree of hydrolysis, e.g. a degree of hydrolysis of 15% or above, for example 20-30%, in order to prepare whey protein hydrolysates with a low antigenicity or hydrolysates that are well-absorbed over the intestine. However, existing whey protein hydrolysates have the problem that when the hydrolysis becomes too extensive and a high degree of hydrolysis is obtained, the whey protein hydrolysate has a bitter unpleasant taste and is therefore not suitable to be used in food products or beverages in large amounts.

WO 02/19 837 A1 discloses a process of preparing a whey protein hydrolysate from a whey protein isolate (WPI) substrate having improved flavour, functionality and ACE-I inhibiting properties. WO 02/19 837 A1 discusses the problems of bitter flavours in whey protein hydrolysates and solve the problem of bitter flavours by controlling the enzymatic hydrolysis such that hydrolysis is terminated when the degree of hydrolysis is maximum 10%, such as 3-10%. WO 2016/156077 relates to milk based protein hydrolysates using microbial or plant sourced enzymes. RU 2 529 707 discloses a whey protein hydrolysate with a high degree of hydrolysis and a low degree of antigenicity.

Hence, a whey protein hydrolysate having a high degree of hydrolysis (a degree of hydrolysis of at least 20%) and hence a low antigenicity and improved absorption properties, but at the same time does not have an unpleasant bitter taste would be advantageous.

**Summary of the invention**

The inventors of the present invention have surprisingly found out that using specific combinations of enzymes for enzymatic hydrolysis of whey protein, whey protein hydrolysates are obtained with a high degree of hydrolysis, while the whey protein hydrolysates have an acceptable taste and no bitter flavour or at least an acceptable level of bitter compounds.

Thus, an object of the present invention relates to a method of preparing a whey protein hydrolysate having a degree of hydrolysis of at least 20% and where a 4% w/w protein solution has a bitterness score corresponding to a solution of 0.08% w/v caffeine or less, without the whey protein hydrolysate being subjected to any bitterness reducing treatments.

The method of the present invention relates to a method of preparing a whey protein hydrolysate that has a low turbidity, and hence is clear in appearance, without any ultrafiltration step.

In particular, it is an object of the present invention to provide a whey protein hydrolysate that solves the above mentioned problems of the prior art with unpleasant bitter taste when the degree of hydrolysis is high.

Thus, one aspect of the invention relates to a method of preparing a whey protein hydrolysate comprising:

a) providing a whey protein solution comprising whey protein in an amount of at least 50% by weight based on the total solid content,
b) subjecting the whey protein solution to enzymatic hydrolysis, wherein the enzymatic hydrolysis is performed with the use of either one of the following enzyme combinations:

i. comprising at least a serine endopeptidase from *Bacillus,* at least a serine endopeptidase from *Aspergillus,* and at least a trypsin-like protease
ii. comprising at least a serine endopeptidase from *Bacillus,* at least a serine endopeptidase from *Aspergillus,* and at least a leucyl aminopeptidase from *Aspergillus*
iii. comprising at least a bacillolysin from *Bacillus amyloliquefaciens,* at least bromelain, and at least a leucyl aminopeptidase from *Aspergillus*

c) stopping the enzymatic hydrolysis by inactivating the enzymes when the degree of hydrolysis (DH) is 20% or more to obtain a whey protein hydrolysate;

wherein the method does not comprise any step of ultrafiltration of the whey protein hydrolysate obtained in step c).

Another aspect of the present invention relates to a whey protein hydrolysate comprising:

- free amino acids and peptides, and
- having a degree of hydrolysis of at least 20%, and
- peptides having a molecular weight of 2500 Da or more in an amount of 8 to 25% by

weight or less of the total amount of peptides, and

- free amino acids in an amount of 15% by weight or less of the total amino acid content in the hydrolysate, and

wherein the whey protein hydrolysate in a 4% w/w protein solution has a bitterness score corresponding to a solution of 0.08% w/v or less of caffeine.

A further aspect of the present invention is to provide a carbonated beverage comprising the whey protein hydrolysate according to the invention.

Yet another aspect not covered by the invention is to provide a beverage comprising the whey protein hydrolysate according to the invention, wherein the whey protein hydrolysate is present in the beverage in an amount corresponding to 2 to 25% by weight hydrolysed whey protein.

Still another aspect of the invention is the use of the whey protein hydrolysate according to the invention as a food ingredient.

**Brief description of the figures**

Figure 1 A shows the nephelometric turbidity (NTU) at different protein concentrations for sample 16 (a whey protein hydrolysate according to the invention made by enzymatic hydrolysis of a WPI), sample 14 (whey protein hydrolysate according to the invention made by enzymatic hydrolysis of a whey protein concentrate (WPC)) and sample 13 (a reference whey protein hydrolysate which is not prepared by using an enzyme combination of the invention, but the whey protein hydrolysate is subjected to ultrafiltration.

Figure 1 B shows the nephelometric turbidity (NTU) at different protein concentrations for sample 16 (a whey protein hydrolysate according to the invention made by enzymatic hydrolysis of a WPI). Standard deviations are given.

Figure 1C shows the nephelometric turbidity (NTU) at different protein concentrations for sample 14 (whey protein hydrolysate according to the invention made by enzymatic hydrolysis of a WPC). Standard deviations are given.

Figure 2 shows samples of different protein concentrations of sample 13, 14 and 16. The concentration of protein (w/w) is from left to right: 8%, 6.4%, 4.8%, 3.2% and 1.8%. A) shows sample 16, B) shows sample 14 and C) shows sample 13.

Figure 3 shows the bitterness score of different concentrations of caffeine and the bitterness score of sample 13, 14 and 16.

Figure 4 shows a spider web representation of the taste and mouthfeel profile of sample 13, 15 and 16. The level of significance of the difference between attributes with the highest and lowest score is indicated by *** and is 99.9% and with $P < 0.001$ (ANNOVA analysis).

Figure 5 shows the percentage of the peptides between 7 and 19 amino acids in the ranges 7-10 amino acids and 11-19 amino acids when analyzed using size exclusion chromatography (SEC) or LC-MS/MS (MS).

Figure 6 shows phenylalanine comprising peptides shown as the percentage of all peptides from alpha-lactalbumin, beta-lactoglobulin and beta-casein. The shortest peptides analyzed were 5 amino acids.

Figure 7 shows the number percentage of peptides of 5-19 amino acids from alpha-lactalbumin, beta-lactoglobulin and beta-casein.

Figure 8 shows samples of beverages from left to the right: a beverage prepared with no heat treatment, a beverage prepared with direct UHT treatment at 143°C for 6 seconds, a beverage prepared with indirect UHT treatment for 6 seconds, and a beverage prepared with pasteurization at 90 °C for 6.5 minutes.

Figure 9 shows pictures of SDS-page gels used to determine the amount of non-degraded BSA in different samples of whey protein hydrolysates.

Figure 10 shows the correlation between carbonation and pH of a beverage.

Figure 11 shows the pH of different samples comprising whey protein hydrolysates and carbonated with 2.5 volumen carbon dioxide per volume of the composition.

Figure 12 shows the pH and turbidity during heating of a sample comprising a solution of 8% protein of sample 16 carbonated with 2.5 volume CO2 per volume solution .

The present invention will now be described in more detail in the following.

**Detailed description of the invention**

Definitions

Prior to discussing the present invention in further details, the following terms and conventions will first be defined: All references to singular characteristics or limitations of the present invention shall include the corresponding plural characteristic or limitation, and *vice versa,* unless otherwise specified or clearly implied to the contrary by the context in which the reference is made.
All percentages referred to herein are percentages by weight unless otherwise stated. Also, the terms "by weight of dry matter" and "on dry matter basis" refer to the same concept and are used interchangeably.
The term "w/w" as in for example 1% w/w refers to a composition comprising 1% by weight of a compound.
The term "palatable" refers to having a taste being good enough for eating and/or drinking, i.e. having an acceptable or satisfactory taste for the human consumer.
Unless defined otherwise, all technical and scientific terms used herein have the same meaning as commonly understood by one of ordinary skill in the art.

Whey protein solution:

In the context of the present invention, the term "solution" as in "whey protein solution" encompass compositions that contain a combination of liquid and solid compounds or semi-solid particles such as e.g. protein particles. A "solution" may therefore be a suspension or even a slurry. However, the "whey protein solution" is preferably pumpable and the amount of liquid in the whey protein solution is preferably 70-98%, more preferably 80-96%. The liquid used for the whey protein solution is typically water.
The whey protein solution will typically comprise protein in an amount of 2% by weight or more of the whey protein solution. In an embodiment of the invention, the whey protein solution comprises protein in the range from 2 to 20% by weight of the whey protein solution. Preferably, the whey protein solution comprises protein in an amount in the range of from 5 to 15% by weight of the whey protein solution.
The whey protein solution used in the hydrolysis is obtained by dispersing a composition comprising whey protein in a liquid, such as water. Preferably, the whey protein solution is made by mixing any of a milk serum protein concentrate, a whey protein concentrate, a milk serum protein isolate and/or a whey protein isolate with water. Thus, in an embodiment of the present invention, the whey protein solution comprises a milk serum protein concentrate, a whey protein concentrate, a milk serum protein isolate and/or a whey protein isolate.
The whey protein solution of the present invention comprises whey protein in an amount of at least 50% based on the total solid content. If the content of whey protein is less than 50% of the total solid content, the overall molecular composition (ratios between protein, carbohydrates, lipids and minerals) will be different, the enzymes may behave differently and hence the product obtained will be different.
Besides from whey protein, the whey protein solution may comprise other proteins in small amounts, for example casein.
The whey protein solution typically comprises other components in addition to protein. The whey protein solution may comprise other components that are normally found in whey or milk serum, such as e.g. minerals, carbohydrates, and/or lipids. Alternatively or additionally, the whey protein solution may comprise components that are not native in the whey or milk serum. However, such non-native milk components should be suitable and safe for use in food production.

The lower the content of protein, based on the total solid content, is in the whey protein solution, the higher is the amounts of lipids, carbohydrates (mainly lactose) and other proteins than whey protein.

The whey protein solution may for example comprise carbohydrates, such as e.g. lactose, oligosaccharides and/or hydrolysis products of lactose (i.e. glucose and galactose). The whey protein solution may e.g. comprise carbohydrate in the range of from 0 to 10 % by weight based on the total solid content.

A whey protein isolate (WPI) and milk serum protein isolate (SPI) comprises very low amounts of carbohydrates, such as lactose. Hence, when a WPI or SPI is used for preparation of the whey protein solution, the content of carbohydrates in the whey protein solution is in the range of from 0 to 1% by weight based on the total solid content. If a whey protein concentrate (WPC) or milk serum protein concentrate (SPC) is used for preparing the whey protein solution, the amount of carbohydrates in the whey protein solution are preferably in the range of from 2 to 8% by weight based on the total solid content.

The whey protein solution may also comprise lipids, e.g. in the form of triglyceride and/or other types of lipids, such as phospholipids.

In the context of the present invention, the terms "fat" and lipid" have the same meaning and may be used interchangeably.

The whey protein solution according to the present invention comprises whey protein in an amount of at least 50% based on the total solid content.

If the protein content in the whey protein solution is less than 50% of the total solid content, the whey protein hydrolysate obtained after hydrolysis may not have the characteristics defining the whey protein hydrolysate according to the present invention. i.e. no unpleasant bitter taste in a 4% protein solution, a degree of hydrolysis above 20%, free amino acids in an amount of 15% by weight or less and peptides having a molecular weight of 2500 Da or more being in an amount of 8 to 25% by weight or less of the total amount of peptides.

A whey protein solution with a whey protein content below 50% based on total solid content will comprise a high amount of minerals, fat and carbohydrates. It is not desired to make a whey protein hydrolysate with a content of whey protein below 50% by weight of the solid content and high amounts of minerals, fats and carbohydrates. Without being bound by any theory, the inventors of the present invention believe that the minerals may affect the activity of some enzymes. This could also apply for lipids and carbohydrates. High amounts of lipids, minerals and carbohydrates may also affect the taste and turbidity of the whey protein hydrolysate obtained.

Preferably, the whey protein solution comprises whey protein in an amount of at least 60% by weight based on the total solid content, such as at least 70% by weight based on the total solid content, even more preferably at least 80% by weight based on the total solid content. In a more preferred embodiment of the present invention, the whey protein solution comprises whey protein in an amount of at least 85% by weight based on the total solid content, most preferred is that the whey protein solution comprises whey protein in an amount of at least 90% by weight based on the total solid content.

The protein present in the whey protein solution should mainly be whey proteins. However, minor amounts of other proteins, such as for example casein, may be present. In an embodiment of the present invention, the whey protein solution therefore comprises whey protein in an amount of 90% by weight or more based on the total amount of protein. Preferably, the whey protein solution comprises whey protein in an amount of 95% by weight or more based on the total amount of protein. Hence, in further embodiments of the present invention, the whey protein solution comprises at most 10% by weight casein or other non-whey protein based on the total amount of protein, preferably at most 5% by weight, more preferably at most 3% by weight casein or other non-whey protein based on the total amount of protein.

If the fat content in the whey protein solution is high, it will influence the clarity and taste of the protein hydrolysate obtained. Hence, in an embodiment of the invention, the whey protein solution comprises lipids in an amount of at most 10% by weight based on the total solid content, such as at most 8% by weight based on the total solid content, even more preferably the whey protein solution comprises lipids in an amount of at most 6% by weight based on the total solid content.

If a whey protein concentrate is used for preparing the whey protein solution, the lipid/fat content is approximately 6-8% by weight of the total solid content. If a whey protein isolate, on the contrary, is used for the preparation of the whey protein solution, the whey protein solution is essentially free of fat.

In a preferred embodiment of the invention, the whey protein solution is essentially free of fat. By the term "essentially free of fat" is meant that the lipid content in the whey protein solution is less than 1% by weight based on the total solid content, preferably less than 0.5% by weight and even more preferably less than 0.1% by weight based on the total solid content.

If the fat content in the whey protein solution is low, such as maximum 0.5% based on the total solid content, the whey protein hydrolysate prepared according to the method of the present invention will be clear in appearance. Preferably, the lipid content in the whey protein solution is less than 0.3% by weight of the total solid content, and more preferably less than 0.2% lipid by weight based on the total solid content.

In a preferred embodiment of the invention, the whey protein hydrolysate is obtained by using a whey protein solution of a whey protein isolate and/or a milk serum protein isolate. When a whey protein isolate and/or a milk serum protein isolate is used for hydrolysis, the fat content will be low (below 0.5%). This makes it possible to prepare a whey protein hydrolysate that besides from having a high degree of hydrolysis (DH>15%) and a good taste, also is clear in appearance, without any ultrafiltration step. Preferably, the whey protein solution is a whey protein isolate or milk serum protein isolate mixed in water.

Whey protein:

In an aspect of the present invention, the whey protein hydrolysate is obtained by hydrolysis of a solution comprising whey protein.

In the context of the present invention, the term "whey protein" pertains to protein that is found in whey or in milk serum. The whey protein of the whey protein solution may be a subset of protein species found in whey or milk serum or it may be the complete set of protein species found in whey and/or in milk serum. Whey protein is a mixture of globular proteins isolated from whey, the liquid material created as a by-product of cheese production. Whey proteins are proteins present in the serum phase of either milk or coagulated milk. The proteins of the serum phase of milk are besides from whey proteins also sometimes referred to as milk serum proteins.

The term "milk serum" pertains to the liquid which remains when casein and milk fat globules have been removed from milk, e.g. by microfiltration or large pore ultrafiltration. Milk serum may also be referred to as "ideal whey".

The term "milk serum protein" or "serum protein" pertains to the protein which is present in the milk serum.

The term "whey" pertains to the liquid supernatant that is left after the casein of milk has been precipitated and removed. Casein precipitation may e.g. be accomplished by acidification of milk and/or by use of rennet enzyme.

Several types of whey exist such as sweet whey, acid whey and casein whey.

The whey protein present in the whey protein solution of the present invention can be derived from different sources of whey, for example casein whey, acid whey or sweet whey.

In a preferred embodiment of the invention, the whey proteins in the whey protein solution is from sweet whey. Sweet whey comprises primarily the proteins beta-lactoglobulin (BLG), alpha-lactalbumin (ALA) and caseinomacropeptide (CMP). However, sweet whey may comprise other proteins, such as immunoglobulins, osteopontin, lactoferrin and fat globule membrane proteins. CMP is not present in casein whey or acid whey. In an embodiment of the invention, the whey proteins in the whey protein solution is from sweet whey where CMP has been fully or partially removed. This may be referred to as modified sweet whey. Removal of CMP from sweet whey results in a protein material with threonine and tryptophan contents that are closer to that of human milk.

In the context of the present invention, the term "beta-lactoglobulin" may also be referred to as "BLG". The terms may be used interchangeably and pertains to BLG from mammal species. Furthermore, the term "alpha-lactalbumin" may in the context of the present invention be referred to as "ALA" and pertains to alpha-lactalbumin from mammal species.

The term "sweet whey" as used herein refers to the liquid remaining after milk has been curdled and strained during the making of rennet type cheeses. "Sweet whey" is obtained during the production of rennet type hard cheese like Cheddar or Swiss cheese. Sweet whey is obtained by adding rennet enzymes to a milk composition, which cleaves kappa-casein into para-kappa-casein and the peptide caseinomacropeptide (CMP), thereby destabilising the casein micelles and causing casein to precipitate. The liquid surrounding the rennet precipitated casein is referred to as sweet whey. The pH value of sweet whey can range between 5.2 and 6.7.

Sweet whey is a product from cheese production that comprises about 10-15% by weight protein and about 75-80% lactose. The proteins in sweet whey are mainly whey proteins, but small amounts of casein may also be present. Whey proteins include beta-lactoglobulin (about 55-65%), alpha-lactalbumin (about 18-25%), bovine serum albumin, immunoglobulins, caseinomacropeptides (CMP), osteopontin, lactoferrin and milk fat globule membrane proteins.

The term "casein whey" (may also sometimes be referred to as sour whey or acid whey) relates to whey, which is obtained from casein/caseinate production. In the context of the present invention, the casein whey is not the same as acid whey. Casein whey is the whey fraction obtained after separation of casein/caseinates by microfiltration. Casein whey does not comprise CMP.

The term acid whey is used for the whey obtained during the production of acid type cheeses such as cottage cheese and quark. In the preparation of acid type cheeses, casein is removed from milk by acid precipitation, i.e. reducing the pH value of the milk to a pH below 4.6 which is the isoelectric point of casein and which causes the casein micelles to disintegrate and precipitate. The pH is often reduced to a range from 3.8 to 4.6. The liquid surrounding the acid precipitated casein is often referred to as acid whey and does not contain CMP.

In an embodiment of the invention, the whey protein used in the whey protein solution is not acid whey or casein whey.

The whey protein used in the whey protein solution in the present invention may be a whey protein concentrate (WPC), a milk serum protein concentrate (SPC), a whey protein isolate (WPI) or a milk serum protein isolate (SPI). The difference between a whey protein concentrate and a whey protein isolate is the composition of the product, particularly the protein content. Whey protein isolates are more pure than concentrates and other non-protein components have been partially removed to "isolate" the whey protein. Thus, a whey protein isolate is having a higher percentage of protein and can be pure enough to be virtually lactose free, carbohydrate free, fat free, and cholesterol free.

In the present context, the terms "whey protein concentrate (WPC)" and "serum protein concentrate (SPC)" encompass both dry and liquid compositions of whey protein. The protein content in a WPC and SPC used in the present invention is not less than 50% by weight based on the total solid content. However, a whey protein concentrate may comprise higher amounts of whey protein, for example 80% by weight whey protein based on the dry matter content. The dry portion of liquid whey is obtained by the removal of sufficient non-protein constituents from whey so that the dry product comprises not less than 50% by weight whey protein.

A WPC or SPC used in the present invention typically comprises:

50-89% by weight protein relative to the total solid content
15-70% by weight BLG relative to the total protein content
8-50% by weight ALA relative to the total protein content
0-40% by weight CMP relative to the total protein content.

Alternatively, but also preferred is a WPC or a SPC comprising"

50-89% by weight protein relative to the total solid content
15-80% by weight BLG relative to the total protein content
4-50% by weight ALA relative to the total protein content
0-40% by weight CMP relative to the total protein content.

Preferably, A WPC or an SPC comprises:

50-89% by weigh protein relative to the total solid content
15-80% by weight BLG relative to the total protein content
4-50% by weight ALA relative to the total protein content
0-40% by weight CMP relative to the total protein content.

More preferably, a WPC or a SPC comprises:

70-89% by weight protein relative to the total solid content
30-80% by weight BLG relative to the total protein content
4-35% by weight ALA relative to the total protein content
0-25% by weight CMP relative to the total protein content.

The terms "whey protein isolate" and "serum protein isolate" pertains to dry or liquid compositions, which generally are considered almost free of lactose and cholesterol and has a whey protein content of at least 90% by weight based on the total solid content. A whey protein isolate may for example comprise 92% by weight whey protein or higher based on the total solid content. Preferably, the WPI and SPI comprises from 90-100% by weight protein based on the total solid content, such as from 92-99% by weight protein based in the total solid content.

A WPI or a SPI may preferably comprise:

90-100% by weight protein relative to the total solid content
15-70% by weight BLG relative to the total protein content
8-50% by weight ALA relative to the total protein content
0-40% by weight CMP relative to the total protein content.

Alternatively, but also preferred, a WPI or a SPI may comprise:

90-100% by weight protein relative to the total solid content
30-80% by weight BLG relative to the total protein content
4-35% by weight ALA relative to the total protein content
0-25% by weight CMP relative to the total protein content.

Preferred, a WPI may preferably comprise:

90-100% by weight protein relative to the total solid content
60-70% by weight BLG relative to the total protein content
10-20% by weight ALA relative to the total protein content
10-20% by weight CMP relative to the total protein content.

In an embodiment of the invention, the whey protein solution used in the preparation of whey protein hydrolysate according to the invention comprises a total amount of whey protein in the range of 50-98% by weight of dry matter, such as 70-97% by weight, preferably 72-95% by weight, even more preferably in the range of 75-95% by weight of dry matter.

Any suitable whey protein source may be used to prepare the whey protein solution according to the present invention. The whey proteins used in whey protein solution according to the present invention is preferably whey proteins from mammalian milk, such as e.g. milk from cow, sheep, goat, buffalo, camel, llama, mare, horse and/or deer. In some preferred embodiments of the invention, the whey proteins are derived from bovine (cow) milk.

It is preferred that the whey protein solution is a demineralised whey protein solution. It is preferred that the content of minerals in the whey protein solution is low, since low concentrations of minerals such as sodium, calcium, potassium magnesium and phosphate are preferred in protein hydrolysates from a nutrition and health perspective. Furthermore, minerals including e.g. sodium and calcium may interact with the whey proteins and affect product turbidity, aggregation behaviour and heat tolerance of whey protein hydrolysates. At high concentration, some minerals including especially sodium, calcium and zinc may inhibit or promote the proteolytic activity of some proteases and thus the presence of high concentration of these ions may change the concerted cleavage pattern of the proteases. Thus, in an embodiment of the invention, the content of minerals in the whey protein solution is 10% or less based on the total solid content, more preferably 8% or less. In the context of the present invention, the term "minerals" refers to the ash content. The terms "mineral" and "ash" may be used interchangeably and refer to the same. Hence, referring to the mineral conent of the whey protein solution, is to be understood as the ash content of the whey protein solution.

In an embodiment of the invention, the whey protein solution comprises 30% by weight or more of BLG of the total protein content, such as 40% by weight or more of BLG. Most preferably, the whey protein solution comprises 50% by weight or more of BLG based on the total protein content, even more preferably the whey protein solution comprises BLG in an amount of 55% by weight or more based on the total protein content. In another embodiment of the invention, the whey protein solution comprises BLG in an amount in the range of from 30 to 95% by weight BLG based on total protein content, such as from 40 to 90% by weight BLG based on total protein content, even more preferably from 45 to 80% by weight based on total protein content.

In the context of the present invention, the term "whey" relates to the liquid composition which is left when casein has been removed from milk. Casein may e.g. be removed by microfiltration providing a liquid permeate which is free or essential free of micellar casein but contains the native whey proteins. This liquid permeate is sometimes referred to as ideal whey, serum or milk serum.

The protein of the whey protein solution is preferably as close to its native state as possible and have preferably only been subjected to gentle heat-treatment if any at all.

Enzymatic hydrolysis:

Step b) in the method according to the present invention relates to subjecting the whey protein solution to enzymatic hydrolysis, wherein the enzymatic hydrolysis is performed with the use of either one of the following enzyme combinations:

i. an enzyme combination comprising at least a serine endopeptidase from *Bacillus,* at least a serine endopeptidase from *Aspergillus,* and at least a trypsin-like protease
ii. an enzyme combination comprising at least a serine endopeptidase from *Bacillus,* at least a serine endopeptidase from *Aspergillus,* and at least a leucyl aminopeptidase from *Aspergillus*
iii. an enzyme combination comprising at least a bacillolysin from *Bacillus amyloliquefaciens,* at least a leucyl aminopeptidase from *Aspergillus* and at least bromelain.

The inventors of the present invention have surprisingly found out that enzymatic hydrolysis of a whey protein solution by addition of any of the three mentioned enzyme combinations, will result in whey protein hydrolysates having a degree of hydrolysis above 20%, peptides having a molecular weight of 2500 Da or nore in an amount of 8 to 25% by weight of the total amount of peptides, a content of free amino acids of 15% or less, and where the whey protein hydrolysate has an acceptable taste and a low content of bitter peptides. The inventors have found out that the whey protein hydrolysates prepared by hydrolysis with the mentioned enzyme combinations i. to iii. have no bitter taste in a 4% w/w protein solution.

In step c) of the present invention, the enzymatic hydrolysis is stopped by inactivating the enzymes when the degree of hydrolysis (DH) is 20% or more to obtain a whey protein hydrolysate. The degree of hydrolysis (DH) is defined as the percentage of peptide bonds in the original proteins that have been cleaved by hydrolysis.

In an embodiment of the invention, the serine endopeptidase from *Aspergillus* is a serine endopeptidase from *Aspergillus oryzae* and/or *Aspergillus flavus.* The serine endopeptidase from *Aspergillus* is preferably a subtilisin-like serine endopeptidase (EC 3.4.21).

In an embodiment of the invention, the enzyme combination i) further comprises a metallo endopeptidase from *Bacillus,* such as bacillolysin. Hence, in an embodiment, enzyme combination i) comprises:

- at least a serine endopeptidase from *Bacillus,* at least a metallo endopeptidase from *Bacillus,* at least a serine endopeptidase from

*Aspergillus,* and at least a trypsin-like protease

In a further embodiment of the invention, the enzyme combination ii) may further comprise a metallo endopeptidase from *Bacillus,* such as bacillolysin. Hence, in an embodiment, enzyme combination ii) comprises:

- at least a serine endopeptidase from *Bacillus,* at least a metallo endopeptidase from *Bacillus,* at least a serine endopeptidase from *Aspergillus,* and at least a leucyl aminopeptidase from *Aspergillus*

In an embodiment of the invention, the leucyl aminopeptidase from *Aspergillus* is from *Aspergillus oryzae.*

In an embodiment, the serine endopeptidase from *Bacillus* is subtilisin. Subtilisin is preferably from *Bacillus licheniformis.*

In another embodiment of the invention, the metallo endopeptidase is bacillolysin. The bacillolysin is preferably from *Bacillus,* and is more preferably from *Bacillus amyloliquefaciens.* In a preferred embodiment of the invention, bacillolysin is not from *Bacillus subtilis.*

In the context of the present invention, the term "trypsin-like protease" is a protease of microbial origin. Preferably, the trypsin-like protease of microbial origin is from *Fusarium sp,* in particular *Fusarium oxsporum.* Hence, the term "trypsin-like protease", for example, does not include pancreatin that is not of microbial origin. On the contrary, pancreatin is an mixture of enzymes derived from the pancreas that for example comprises trypsin, chymotrypsin, amylase and lipase. Further, the term "trypsin-like protease" should not be confused with "trypsin".

In still another embodiment, the bromelain is from *Ananas comosus.* Bromelain is a cysteine endopeptidase.

In an embodiment of the invention, the enzymatic hydrolysis in step b) is performed with the use of either one of the following enzyme combinations:

i) an enzyme combination comprising at least a serine endopeptidase from *Bacillus,* at least a serine endopeptidase from *Aspergillus,* and at least a trypsin-like protease,
ii) an enzyme combination comprising at least a serine endopeptidase from *Bacillus,* at least a serine endopeptidase from *Aspergillus,* and at least a leucyl aminopeptidase from *Aspergillus,*
iii) an enzyme combination comprising at least a combination of a serine endopeptidase and metallo endopetidase from *Bacillus,* at least a serine endoprotease from *Aspergillus,* and at least a leucyl aminopeptidase from *Aspergillus,*
iv) an enzyme combination comprising at least a bacillolysin from *Bacillus amyloliquefaciens,* at least bromelain, and at least a leucyl amino peptidase from *Aspergillus.*

In a preferred embodiment of the invention, the enzymatic hydrolysis in step b) is performed with the use of either one of the following enzyme combinations:

i) an enzyme combination comprising at least a serine endopeptidase from a *Bacillus* species, at least a serine endopeptidase from *Aspergillus oryzae,* and at least a trypsin-like protease of microbial origin,
ii) an enzyme combination comprising at least subtilisin from a *Bacillus* species, at least a serine endopeptidase from *Aspergillus oryzae,* and at least a leucyl aminopeptidase from *Aspergillus oryzae,*
iii) an enzyme combination comprising at least a combination of bacillolysin and subtilisin from a *Bacillus* species, at least a serine endoprotease from *Aspergillus oryzae,* and at least a leucyl aminopeptidase from *Aspergillus oryzae,*
iv) an enzyme combination comprising at least a bacillolysin from *Bacillus amyloliquefaciens,* at least bromelain from *Ananas comosus,* and at least a leucyl amino peptidase from *Aspergillus oryzae.*

In preferred embodiments of the invention, the enzyme combinations are:

i) enzyme combination comprising at least an enzyme from the group of EC 3.4.21.62, at least a further enzyme of the group EC 3.4.21, and at least a further enzyme of the group of EC 3.4.21.4,
ii) an enzyme combination comprising at least an enzyme from the group of EC 3.4.21.62, at least a further enzyme of the group of EC 3.4.21, and at least a further enzyme of the group of EC 3.4.11,
iii) an enzyme combination comprising at least an enzyme from the group of EC 3.4.21.62, a further enzyme from the group of EC 3.4.24.28, at least a further enzyme of the group of EC 3.4.21 and at least a further enzyme of the group of EC 3.4.11,
iv) an enzyme combination comprising at least an enzyme from the group of EC 3.4.24.28, at least a further enzyme from the group of EC 3.4.22.32 and at least a further enzyme of the group of EC 3.4.11.

In another preferred embodiment of the invention, the enzyme combinations are:

i) comprising at least a serine endopeptidase from *Bacillus licheniformis,* at least a serine endopeptidase from *Aspergillus oryzae,* and at least a trypsin-like protease from *Fusarium oxysporum,* optionally also bacillolysin from *Bacillus amyloliquefaciens,*
ii) comprising at least a serine endopeptidase from *Bacillus licheniformis,* at least a serine endopeptidase from *Aspergillus oryzae* and at least a leucyl aminopeptidase from *Aspergillus oryzae,*
iii) comprising at least a serine endopeptidase from *Bacillus licheniformis,* a bacillolysin from *Bacillus amyloliquefaciens,* at least a serine endopeptidase from *Aspergillus* oryzae and at least a leucyl aminopeptidase from *Aspergillus oryzae,*
iv) comprising at least a bacillolysin from *Bacillus amyloliquefaciens,* at least bromelain from *Ananas comosus* and at least a leucyl aminopeptidase from *Aspergillus* oryzae.

The serine endopeptidase from *Bacillus licheniformis* is preferably subtilisin.
In another preferred embodiment of the invention, the enzyme combinations are:

i) comprising at least a serine endopeptidase from *Bacillus* (EC 3.4.21.62), at least a serine endopeptidase from *Aspergillus* (EC 3.4.21), and at least a trypsin-like protease (EC 3.4.21.4),
ii) comprising at least a serine endopeptidase from *Bacillus* (EC 3.4.21.62), at least a serine endopeptidase from *Aspergillus* (EC 3.4.21) and at least a leucyl aminopeptidase from *Aspergillus* (EC 3.4.11),
iii) comprising at least a serine endopeptidase from *Bacillus* (EC 3.4.21.62), a bacillolysin (EC 3.4.24.28), at least a serine endopeptidase from *Aspergillus* (EC 3.4.21.63,) and at least a leucyl aminopeptidase from *Aspergillus* (EC 3.4.11),
iv) comprising at least a bacillolysin from *Bacillus amyloliquefaciens* (EC 3.4.24.28), at least bromelain [EC 3.4.22.32], and at least a leucyl aminopeptidase from *Aspergillus* (EC 3.4.11).

The enzyme combinations i) to iv) used in the present method of preparing a whey protein hyrolysate may comprise other enzymes than the primary enzymes mentioned. By the term "primary enzymes" is meant the enzymes that are most abundant in the preparations. In the following listing, Uniprot accession numbers are given in parentheses to identify the proteases annotated to a specific naming.
For example, the enzyme combinations may comprise one or more enzymes with high sequence identity (95-100%) to enzymes selected from the group consisting of peptide hydrolase (A0A364MDR7), subtilase family protein (I8A6W5), fungalysin metallopeptidase M36 (A0A2P2H013), neutral protease 2 (A0A364MH70), aspergillopepsin-1 (B8NLY9), leucyl aminopeptidase A (Q2U1F3), leucyl aminopeptidase 2 (Q2ULM2), dipeptyl peptidase 4 (Q2UH35), dipeptidyl peptidase 5 (Q9Y8E3), neutral protease 1 (Q2U1G7), neutral protease 2 (P46076), alkaline protease 1 (P12547), and prolyl oligopeptidase family protein (B8NBM3).
In an aspect of the invention, the primary enzymes of enzyme combination i) are serine endopeptidase from *Bacillus,* a serine endopeptidase from *Aspergillus* and a trypsin-like protease. The serine endopeptidase from *Bacillus* is preferably subtilisin, and the serine endopeptidase from *Aspergillus* is preferably subtilase family protein. In an embodiment of the invention, enzyme combination i) may further comprise one or more of aminopeptidase (such as peptide hydrolase, and leucyl aminopeptidase), metallo endopeptidase (such as bacillolysin and fungalysin metallopeptidase, neutral protease), prolyl oligopeptidase family protein, and aspergillopepsin-1 (aspartic endopeptidase). It is expected that at least 80% of the enzymes present in enzyme combination i) are serine endopeptidase from *Bacillus,* serine endopeptidase from *Aspergillus* and trypsin-like protease. An example of a preparation comprising a serine endopeptidase from *Bacillus* is Protamex (Novozymes A/S). Protamex also comprises bacillolysin. Examples of preparations comprising a serine endopeptidase from *Aspergillus* are Promod 782 (Biocatalysts Ltd) and Protease A Amano 2 SD (Amano Enzyme Ltd), where the primary enzyme is a serine endopeptidase from *Aspergillus ozyzae.* Promod 782 and Protease A Amano 2 SD also comprise the enzymes peptide hydrolase, leucyl aminopeptidase, fungalysin metallopeptidase M36, prolyl oligopeptidase family protein, neutral protease 2 and aspergillopepsin-1 and the primary enzymes are serine endopeptidases. The trypsin-like protease may for example be provided from Formea TL 1200 BG (Novozymes A/S).

In an aspect of the invention, the enzyme combination ii) comprises serine endopeptidase from *Bacillus,* serine endopeptidase from *Aspergillus* and leucyl aminopeptidase from *Aspergillus* as the primary enzymes. The serine endopeptidase from *Bacillus* is preferably subtilisin, and the serine endopeptidase from *Aspergillus* is preferably subtilase family protein and alkaline protease. The leucyl aminopeptidases from *Aspergillus* may for example be one or more of peptide hydrolase, leucyl aminopeptidase A and leucyl aminopeptidase 2. In an embodiment of the invention, enzyme combination ii) may further comprises one or more of the metallo endopeptidases; fungalysin metallopeptidase M36, neutral protease 1 and neutral protease 2. Enzyme combination ii) may also comprise, aspergillopepsin-1 (aspartic endopeptidase), dipeptyl peptidase 4, and dipeptidyl peptidase 5. It is expected that at least 80% of the enzymes present in enzyme combination ii) are serine endopeptidase from *Bacillus,* serine endopeptidase from *Aspergillus* and leucyl aminopeptidase from *Aspergillus.* An example of a preparation comprising a serine endopeptidase from *Bacillus* is Alcalase (Novozymes A/S) which comprises subtilisin. Examples of preparations comprising a serine endopeptidase from *Aspergillus* are Protease A Amano 2 SD and Promod 782, where the primary enzyme is a serine endopeptidase from *Aspergillus ozyzae.* Promod 782 and Protease A Amano 2 SD also comprise the enzymes peptide hydrolase, leucyl aminopeptidase, fungalysin metallopeptidase M36, prolyl oligopeptidase family protein, neutral protease 2, Aspergillopepsin-1, and the primary enzymes are serine endopeptidases. An example of a preparation comprising leucyl aminopeptidase from *Aspergillus* is Flavourzyme Conc BG (Novozymes A/S) where the primary enzyme is a leucyl aminopeptidase. Flavourzyme Conc BG also comprises leucyl aminopeptidase A, leucyl aminopeptidase 2, dipeptyl peptidase 4, dipeptidyl peptidase 5, neutral protease 1, neutral protease 2, alkaline protease 1 and the primary enzyme is the leucyl aminopeptidases.

In an aspect of the invention, the enzyme combination iii) comprises serine endopeptidase from *Bacillus,* metallo endopeptidase from *Bacillus,* serine endopeptidase from *Aspergillus* and a leucyl aminopeptidase from *Aspergillus* as the primary enzymes. The serine endopeptidase from *Bacillus* is preferably subtilisin, the metallo endopeptidase from *Bacillus* is preferably bacillolysin and the serine endopeptidase from *Aspergillus* is preferably subtilase family protein and alkaline protease. The leucyl aminopeptidases from *Aspergillus* may for example be one or more of peptide hydrolase, leucyl aminopeptidase A and leucyl aminopeptidase 2. In an embodiment of the invention, enzyme combination iii) may further comprise one or more of the metallo endopeptidases; fungalysin metallopeptidase M36, neutral protease 1 and neutral protease 2. Enzyme combination ii) may also comprise, aspergillopepsin-1 (aspartic endopeptidase), dipeptyl peptidase 4 and dipeptidyl peptidase 5. At least 80% of the enzymes present in enzyme combination iii) are serine endopeptidase from *Bacillus,* bacillolysin, serine endopeptidase from *Aspergillus* and a leucyl aminopeptidase from *Aspergillus.* Examples of preparations comprising both serine endopeptidase from *Bacillus* (subtilisin) and bacillolysin are Promod 950L (Biocatalysts Ltd) and Protamex. Examples of preparations comprising a serine endopeptidase from *Aspergillus* are Protease A Amano 2 SD and Promod 782. Promod 782 and Protease A Amano 2 SD also comprise the enzymes peptide hydrolase, leucyl aminopeptidase, fungalysin metallopeptidase M36, prolyl oligopeptidase family protein, neutral protease 2 and aspergillopepsin-1, and the primary enzymes are serine endopeptidases. An example of a preparation comprising leucyl aminopeptidase from *Aspergillus* is Flavourzyme conc BG where the primary enzyme is a leucyl aminopeptidase. Flavourzyme conc BG also comprises the enzymes leucyl aminopeptidase A, leucyl aminopeptidase 2, dipeptyl peptidase 4, dipeptidyl peptidase 5, neutral protease 1, neutral protease 2 and alkaline protease 1, and the primary enzyme is leucyl aminopeptidase.

In an aspect of the invention, the enzyme combination iv) comprises bacillolysin from *Bacillus amyloliquefaciens,* bromelain and leucyl aminopeptidase from *Aspergillus* as the primary enzymes. The leucyl aminopeptidases from *Aspergillus* may for example be one or more of peptide hydrolase, leucyl aminopeptidase A and leucyl aminopeptidase 2. In an embodiment of the invention, enzyme combination iv) may further comprise one or more of the metallo endopeptidases; fungalysin metallopeptidase M36, neutral protease 1 and neutral protease 2. Enzyme combination ii) may also comprise dipeptyl peptidase 4, dipeptidyl peptidase 5 and alkaline protease (serine protease from *Aspergillus*). It is expected that at least 80% of the enzymes present in enzyme combination iv) are bacillolysin from *Bacillus amyloliquefaciens,* bromelain and leucyl aminopeptidase from *Aspergillus.* An example of a preparation comprising bacillolysin from *Bacillus amyloliquefaciens* is Neutrase. An example of bromelain is Promod 523 MDP while an example of leucyl aminopeptidase from *Aspergillus* is Flavourzyme conc BG.

The method of preparing whey protein hydrolysates of the present invention should not be limited to the amount of enzymes added under the hydrolysis step, since the amount of enzyme added is dependent on the type of enzyme and the activity of the enzyme. However, as a guidance the enzymatic hydrolysis is performed with a combination of enzymes, where the total amount of enzymes is in the range of from 0.05 to 10 g per 100 g protein, such as from 0.1 to 7.5 g enzyme per 100 g protein. Preferably, the amount of enzyme is in the amount of from 0.2 to 5.0 g per 100 g protein.

The ratio between the three different enzymes in the combinations i. to iii. may for example be in the range of 1-10:1-10: 1-10, such as in the range of 1-8:1-8:1-8. However, the present invention should not be limited to the amount of enzyme added, since this will be dependent of the activity of the enzymes used.

The enzymatic hydrolysis performed in step b) of the present invention is preferably performed at a temperature in the range of from 40°C to 75°C, such as from 40°C to 70°C. The enzymatic hydrolysis should be performed at a temperature where the enzymes have optimal activity. In a preferred embodiment, the enzymatic hydrolysis is performed at a temperature in the range of 45°C to 65°C.

The time period for the enzymatic hydrolysis before the hydrolysis is stopped in step c) is dependent on the amount and activity of the enzymes used. The hydrolysis is continued until the degree of hydrolysis is 20% or more. The enzymatic hydrolysis in step b) is preferably performed at a time period in the range of 3 hours to 20 hours, such as from 3.5 hours to 15 hours, preferably from 4 hours to 10 hours and even more preferably from 4 hours to 7 hours.

The whey protein solution should preferably have a pH in the range of from 6 to 9 during enzymatic hydrolysis. In a preferred embodiment, the pH during the enzymatic hydrolysis in step b) is from 6.5 to 8.0.

In this pH range the enzymes have most activity and therefore cleaves the proteins to peptides and free amino acids most efficiently. In addition, at this pH range aggregation is avoided, both during the hydrolysis process but also during the heat treatment to inactivate the enzymes.

In step c) of the method of preparing a whey protein hydrolysate according to the present invention, the enzymatic hydrolysis is stopped by inactivating the enzymes. In the context of the present invention, the term "inactivation " refers to irreversible inactivation of the enzyme. The inactivation of enzymes must be irreversible such that the enzymes will not become active under other conditions.

The hydrolysis is stopped when the degree of hydrolysis is at least 20%. In an embodiment of the invention, the hydrolysis is stopped in step c) when the degree of hydrolysis is in the range of from 20% to 35%, preferably from 20% to 30%, and even more preferably from 20% to 28%.

The inactivation of the enzymes in step c) and hence the stopping of the hydrolysis can be by any method known in the art. For example inactivation of the enzymes by amending the temperature to a temperature where the enzymes are inactive and denatured. The inactivation and denaturation of the enzymes could also be by amending the pH of the solution to a pH where the enzymes are inactive.

Hence, in an embodiment of the invention, inactivation of the enzymes in step c) is by heating the whey protein solution with added enzymes to a temperature of at least 80°C. The inactivation of enzymes is preferably by heating to a temperature of from 80°C to 130°C, such as from 85°C to 125°C, even more preferably from 90°C to 120°C. Inactivation of the enzymes in step c) by heating may for example be by heating to a high temperature for a short time period, such as heating to a temperature from 110°C to 130°C for 10 to 30 seconds. Alternatively, the inactivation of the enzymes in step c) may be by heating to a relatively low temperature, but for a longer time period. This could involve heating to from 80°C to 90°C for 5 to 10 minutes.

In another embodiment of the present invention, the irreversible inactivation of enzymes in step c) comprises increasing or decreasing the pH of the whey protein solution with added enzymes, i.e. the whey protein hydrolysate, to a pH where the enzymes are inactive. In an embodiment of the invention, the pH is increased to a pH of 10 or above. In another embodiment, the pH is decreased to a pH of 4 or below.

The method does not comprise any step of ultrafiltration of the whey protein hydrolysate obtained in step c). It was surprising for the inventors of the present invention that enzymatic hydrolysis of a whey protein solution with a low amount of lipids, i.e. a WPI or SPI, with the specified enzyme combinations resulted in whey protein hydrolysates with a palatable taste and which also were clear in appearance without any ultrafiltration steps involved.

Whey protein hydrolysates known in the art can be divided into ultrafiltrated and non-ultrafiltrated hydrolysates. Known non-ultrafiltrated protein hydrolysates will have an unclear or turbid appearance, where the ultrafiltrated protein hydrolysates are generally clear in appearance.

In the context of the present invention, the term "ultrafiltration" refers to membrane filtration with a membrane having a cut off in the range of from 1500 Da to 50000 Da, preferably 2000 Da to 20000 Da.

During ultrafiltration of protein hydrolysates, fat, intact protein as well as some of the larger peptides is retained by the ultrafiltration membrane and retained in the retentate, while free amino acids, smaller peptides and minerals are in the ultrafiltration permeate.

In an embodiment of the present invention, the whey protein solution is prepared as a solution of WPI and SPI with a low lipid content. It was surprising for the inventors of the present invention, that preparing a whey protein hydrolysate with the enzyme combinations of the present invention, resulted in whey protein hydrolysates having a high degree of hydrolysis, a good taste with low bitterness and were clear in appearance.

However, the low lipid content is not the only explanation of why it was possible for the inventors of the present invention to prepare clear protein hydrolysates. The inventors of the present invention surprisingly have found out that when a whey protein solution with a low lipid content was subjected to enzymatic hydrolysis with any one of the mentioned enzyme combinations, it was possible to prepare a whey protein hydrolysate having a degree of hydrolysis above 20%, having no bitter taste in a 4% protein solution and being clear in appearance. To be clear in appearance, it was necessary to use a whey protein solution with a low lipid content. However, the low lipid content was not the only reason why clear hydrolysates were obtained. It was surprisingly found out by the inventors of the present invention that hydrolysis with the specific enzyme combinations lead to clear hydrolysates. In comparative tests, it was observed that other whey protein hydrolysates with a low lipid content but where the hydrolysis was done with other enzymes than the ones used in the method of the present invention, did not result in whey protein hydrolysates with a clear appearance and low bitterness. The whey protein hydrolysate obtained by the method of the present invention may preferably be concentrated and/or dried. Hence, in an embodiment of the invention, the method comprises a step d) of concentrating and/or drying the whey protein hydrolysate obtained in step c). Concentration may for example be by one or more of the unit operations nanofiltration, reverse osmosis filtration, and evaporation.

In another embodiment of the invention, the drying step involves one or more of the unit operations spray drying, freeze drying and spin-flash drying, rotary drying and/or fluid bed drying may also be used.

Whey protein hydrolysate

In an aspect, the present invention relates to a whey protein hydrolysate comprising:

- free amino acids and peptides, and
- having a degree of hydrolysis of at least 20%, and
- peptides having a molecular weight of 2500 Da or more in an amount of 8 to 25% by weight or less of the total amount of peptides, and
- free amino acids in an amount of 15% by weight or less of the total amino acid content in the hydrolysate, and

wherein the whey protein hydrolysate in a 4% protein solution has a bitterness score corresponding to a solution of 0.08% w/v or less caffeine.

In an aspect of the invention, the degree of hydrolysis of the whey protein hydrolysate of the invention is at least 20%. An object of the present invention was to make a whey protein hydrolysate with a high degree of hydrolysis, which without any step of ultrafiltration had no unpleasant bitter taste. It is well known that peptides are responsible for the bitter taste in many hydrolysates. In general, extensive hydrolysis providing hydrolysates with a high degree of hydrolysis is expected to result in hydrolysates with a bitter taste. To reduce the bitter taste of extensively hydrolysed protein hydrolysates, the hydrolysates may be treated with activated charcoal which may be removed again along with bitter tasting peptides. Extensively hydrolysed proteins may be desired because the peptides have other functionalities than intact protein. For example, the peptides may withstand heat treatments better than the intact whey protein.

However, the inventors of the present invention have surprisingly found a method of preparing a whey protein hydrolysate having a high degree of hydrolysis that does not have an unpleasant bitter taste even though the whey protein hydrolysate was not subjected to any additional treatments for removing bitter tasting peptides.

In a preferred embodiment of the invention, the degree of hydrolysis of the whey protein hydrolysate is at least 20%.

In another embodiment of the present invention, the whey protein hydrolysate according to the invention has a degree of hydrolysis from 20 to 35%, such as from 20 to 30% preferably 20 t'& to 28% and even more preferably from 20 to 25%.

The whey protein hydrolysate may comprise free amino acids, and if free amino acids are present, they are present in an amount of 15% by weight or less of the total amino acid content in the hydrolysate. Preferably, the free amino acid content is 12% by weight or less of the total amino acid content. By the term "total amino acid content" is in the context of the present invention meant the total amount of amino acids present, including free amino acids and the amino acids bound in peptides and proteins.

The free amino acid content in the whey protein hydrolysate is not more than 15% by weight of the total amino acid content, such as not more than 13% by weight of the total amino acid content, preferably not more than 10% by weight of the total amino acid content, even more preferably, the content of free amino acids is not more than 8% of the total amino acid content.

In other embodiments of the invention, the whey protein hydrolysate comprises free amino acids in an amount of 2-15% by weight of the total amino acid content in the hydrolysate, preferably free amino acids in an amount of 4-13% by weight of the total amino acid content in the hydrolysate.

The inventors of the present invention have found out that the peptides in the whey protein hydrolysate of the invention comprises peptides having a molecular weight of 2500 Da or more in an amount in the range of from 8 to 25% by weight, even more preferably from 10 to 20% by weight.

In an embodiment of the invention, the whey protein hydrolysate of the invention comprises peptides having a molecular weight of 375 Da or less in an amount of at least 10% by weight. The peptides having a molecular weight of 375 Da or less may for example be present in the whey protein hydrolysate in an amount in the range of from 10 to 25% by weight.

The inventors of the present invention have found out that the whey protein hydrolysates of the invention have a reduced bitter taste as compared to known whey protein hydrolysates having a high degree of hydrolysis, also if those have been subjected to membrane filtration with an ultrafiltration membrane and/or subjected to treatment by activated charcoal.

The bitterness of the whey protein hydrolysates has been compared to the bitterness of caffeine, and the whey protein hydrolysates in a 4% w/w protein solution have a taste less bitter than the taste of a solution of 0.08% w/v caffeine.

Hence, the bitterness score of the whey protein hydrolysates in a 4% w/w protein solution corresponds to the bitterness score of 0.08% w/v caffeine or less. Preferably, the bitterness score of the whey protein hydrolysates in a 4% w/w protein solution of the invention corresponds to the bitterness score of 0.07% w/v caffeine or below, even more preferably to a bitterness score of 0.065% caffeine or below. Most preferably, the bitterness score of the whey protein hydrolysates in a 4% w/w protein solution of the invention corresponds to the bitterness score of 0.060% w/v.

In a further embodiment of the invention, the whey protein hydrolysate has a nephelometric turbidity (NTU) of 100 or below in a 4% w/w protein solution. It is a further embodiment of the invention to obtain a whey protein hydrolysate that besides from having a high degree of hydrolysis and an acceptable taste, is also clear in appearance. A clear and good tasting whey protein hydrolysate is desired, since it can be used in for example beverages, gels, and shakes and give an improved consumer appeal.

If the nephelometric turbidity measured is lower than 100 NTU in a 4% w/w protein solution, the sample is perceived as transparent. If the nephelometric turbidity is above 100 NTU in a 4% protein solution, the measured whey protein hydrolysate is perceived as not being transparent. If the nephelometric turbidity is below below 40 NTU, the solution is perceived as clear. However, a whey protein hydrolysate having a turbidity between 40 and 100 NTU may be transparent (but unclear or turbid). In the context of the present invention, the term "transparent" refers to a solution allowing some light to pass through so that objects behind the solution can be seen, i.e. it is possible to see through the solution. The term "clear" refers to a solution, that is colorless and therefore it is possible to see through the solution with nothing limiting the see through. Hence, a solution may be transparent but not clear.

In a further embodiment of the invention, the whey protein hydrolysate has a nephelometric turbidity (NTU) of 80 or below in a 4% w/w protein solution, such as a nephelometric turbidity (NTU) of 60 or below in a 4% w/w protein solution, preferably a nephelometric turbidity (NTU) of 50 or below in a 4% w/w protein solution, even more preferably a nephelometric turbidity (NTU) of 40 or below in a 4% w/w protein solution.

In also an embodiment of the invention, the whey protein hydrolysate has an antioxidative activity. Preferably the antioxidative activity of the whey protein hydrolysate is measured as having a scavenging percentage of 54 to 60 in a 1.5% by weight protein solution.

The whey protein hydrolysate may comprise other ingredients than protein, for example carbohydrates, lipids and minerals.

In an embodiment of the invention, the whey protein hydrolysate comprises lipids in an amount of 8% by weight or less based of the total solid content, such as 6% by weight or less based of the total solid content. In another embodiment, the whey protein hydrolysate comprises lipids in an amount of 1% by weight or less based on the total solid content, such as a lipid content of 0.5% by weight or less of the total solid content.

The whey protein hydrolysate may also comprise minerals, such as potassium, sodium and calcium.

In an embodiment of the invention, the whey protein hydrolysate comprises potassium in an amount of 3.0% by weight or below.

In another embodiment of the invention, the whey protein hydrolysate comprises sodium in an amount of 2% by weight or below.

In still another embodiment of the invention, the whey protein hydrolysate comprises citric acid in an amount in the range of 4 to 10 g per kg solid content of the whey protein hydrolysate.

In an embodiment of the invention, the whey protein hydrolysate comprises intact or non-degraded bovine serum albumin (BSA) in an amount of 0.5 to 2% by weight based on total protein content.

In a preferred embodiment of the invention, the whey protein hydrolysate is in the form of a dry composition, such as a powder or granulate.

In another embodiment, the whey protein hydrolysate is a liquid composition.

Food products:

An aspect, not covered by the claimed invention relates to providing a food product comprising the whey protein hydrolysate according to the invention.

The food product may for example be any one selected from the group of dairy products, including a beverage, a shake, a gel, a food bar, a concentrate or a liquid shot.

The food product is selected from the group of a protein beverage, a protein shot, a protein shake, a protein gel or a protein bar.

The food product may also be an infant formula or other infant nutrition products. If the food product is in a liquid form, such as a beverage, shake, gel or shot, the food product may comprise the whey protein hydrolysate according to the invention. The whey protein hydrolysate is preferably present in the beverage in an amount corresponding to 2 to 25% by weight hydrolysed whey protein, preferably from 3 to 20% by weight hydroysed whey protein, such as from 3 to 15% by weight hydrolysed whey protein, even more preferably form 3 to 10% by weight hydrolysed whey protein.

If the food product is a bar, such as a protein bar, the food product comprises the whey protein hydrolysate according to the invention in an amount corresponding to 2 to 30% by weight hydrolysed whey protein. Preferably, the amount of whey protein hydrolysate according to the invention is present in a bar in an amount corresponding to from 3 to 20% by weight hydrolysed whey protein, such as from 4 to 15% by weight. If the whey protein hydrolysate according to the invention is used in a food bar, such as for example a protein bar, the whey protein hydrolysate may be used as a softener. It is well-known that increasing concentrations of protein hydrolysate in protein bars have a softening effect and prevents hardening of bars during longer term storage.

A further aspect, not covered by the claimed invention, relates to providing a beverage comprising the whey protein hydrolysate according to the invention in an amount corresponding to 2 to 20% by weight hydrolysed whey protein. The beverage may for example be a protein beverage that besides from protein comprises carbohydrates, vitamins and minerals.

The beverage has a neutral pH, i.e. a pH in the range from 6.5 to 8.0 in a 4% protein solution at 22°C.

In an embodiment of the invention, the whey protein hydrolysate according to the invention may be used as an ingredient in the preparation of a carbonated beverage. Hence, the food product of the invention comprising the whey protein hydrolysate according to the invention is a carbonated beverage.

A further embodiment of the invention relates to a carbonated beverage comprising the whey protein hydrolysate of the invention.

In an embodiment, the carbonated beverage comprises the whey protein hydrolysate in an amount corresponding to 2 to 10% by weight. The carbonated beverage may also comprise carbohydrates, and if carbohydrates are present it is in an amount of 5% by weight or below. The carbonated beverage preferably comprises no fat.

In an embodiment, the amount of carbonation in a carbonated beverage comprising the whey protein hydrolysate of the invention ranges from 0.1 volumes of carbonation (per volume of liquid present in the beverage) to 4 volumes of carbonation. More typically, the amount of carbonation ranges from about 1.6 volumes to about 3.5 volumes, with the most typical concentration ranging from about 1.7 volumes to about 3.0 volumes, and the amount of carbonation is most preferably in the range of from 2.0 to 3.0 volumes. By carbonation is in the context of the present invention meant adding carbon dioxide to a mixture of ingredients for the beverage in an amount sufficient to obtain a carbonated protein beverage where the amount of carbonation present in the beverage ranges from 0.1 volumes to 4 volumes per volume of liquid mixture.

In some embodiments of the method, the carbon dioxide is added in the form of sterile carbonated water. In other embodiments, sterile carbon dioxide is bubbled through the liquid mixture until the desired amount of carbon dioxide is present.

Carbonation increases the acidity of a beverage. The more carbon dioxide added to a beverage, the lower will the pH value of the beverage become. However, the inventors of the present invention have found out that addition of carbon dioxide will decrease pH of the beverage to a minimum of pH 5.5-6.0 at a temperature of 5°C. Adding about 2.5-3.0 volumes carbon dioxide per volume beverage resulted in reduction of pH of the beverage to about pH 6.0 at a temperature of 5°C, while adding about 4 volumes carbon dioxide per volume beverage resulted in reduction of pH of the beverage to about pH 5.5 at a temperature of 5°C.

Hence, in an embodiment of the invention, the carbonated beverage comprising the whey protein hydrolysate of the invention has, at a temperature of 5°C a pH of at least 5.5. The pH will typically be in the range of 5.5 to 8.25, such as in the range of 5.5 to 7.0, preferably a pH in the range of 5.8 to 6.5. A carbonated beverage having a pH above 5.5 is preferred.

The carbonated beverage comprising the whey protein hydrolysate of the invention may for example be heat treated, such as for example pasteurized or autoclaved. The inventors of the present invention have found out that the turbidity of a carbonated beverage comprising the whey protein hydrolysate of the invention does not change after heat treatment at temperatures up to 120°C for a prolonged period of time, such as for example 20 minutes.

In a further embodiment of the invention, a carbonated beverage may comprise the whey protein hydrolysate according to the invention in combination with an unhydrolysed whey protein isolate.

The whey protein hydrolysate of the invention may also be used in the preparation of a protein shot, a protein shake or a protein gel. The protein shot, shake or gel may besides from hydrolysed whey protein in an amount of 2 to 20% by weight comprise carbohydrates, vitamins and minerals. The pH of the protein shot, protein shake or protein gel is preferably neutral, i.e. a pH in the range of from 6.5 to 8.0.

In further aspects, the invention relates to the use of the whey protein hydrolysates according to the invention as a food ingredient. The whey protein hydrolysate may be added to any type of food product as a food ingredient. Preferably, the whey protein hydrolysate of the invention is used as a food ingredient in the preparation of cold or warm beverages.

In an embodiment of the invention, the whey protein hydrolysate is used as a food ingredient in the preparation of UHT stable beverages having a pH in the range from 6.5 to 8.5.

In another embodiment of the invention, the whey protein hydrolysate is used as a food ingredient in the preparation of beverages used for sports nutrition. In the context of the present invention, the term "sports nutrition" refers to nutrition suitable in connection with exercising or training, i.e. for building muscle mass.

In still another embodiment of the invention, the whey protein hydrolysate is used as a food ingredient in the preparation of clinical beverages. In the context of the present invention, the term "clinical beverage" refers to beverages having a clinical or medical indication. For example, a clinical beverage could have a health related effect. A clinical beverage is typically used by hospitalized or elderly people with nutritional difficulties or individuals that require pre-digested protein in order to recover from a medical condition. For example. A clinical beverage could be a beverage used for individuals suffering of malnutrition or malabsorption. The clinical beverage could also be for individuals suffering from gastro-intestinal diseases. In the context of the present context, the term "clinical beverage" and "medical beverage" have the same meaning.

A clinical beverage could for example comprise the whey protein hydrolysate of the invention in an amount corresponding to the beverage comprising from 2 to 20% by weight hydrolysed whey protein. The clinical beverage may comprise carbohydrates in an amount of from 5 to 50% by weight of the beverage. The amount of carbohydrates may for example be in the range of from 10 to 40% by weight, such as from 15 to 35% by weight. The clinical beverage may also comprise fat. For example the fat content in the clinical beverage could be in the range of from 2 to 30% by weight, such as from 3 to 20% by weight, more preferably from 3 to 18% by weight.

In an example, the clinical beverage comprises hydrolysed whey protein according to the invention corresponding to an amount of 4-10% by weight, 3-15% by weight fat and 10-35% by weight carbohydrates.

The clinical beverage preferably has a neutral pH value, i.e. a pH in the range of from 6.5 to 8.0.

The clinical beverage may be in the form of a clear beverage, a milky beverage, a tube feed or in the form of a powder to be reconstituted in a liquid.

In an embodiment, the whey protein hydrolysate of the invention may also be used in the preparation of juice-style beverage. The juice-style beverage preferably comprises the hydrolysed whey protein according to the invention in an amount corresponding to 4-10% by weight protein, 0-1% by weight fat and 15-35% by weight carbohydrates.

If the clinical beverage is in the form of a tube feed, it may comprise from 4 to 15% by weight hydrolysed whey protein according to the invention, about 5-35% by weight carbohydrates and about 3 to 15% by weight fat.

The whey protein hydrolysate according to the present invention may also be used in infant nutrition, such as in infant formulas. In the context of the present invention, the term "infant formula" refers to any type of infant formulas, including follow-on formula, growing-up formula and preterm formula.

If the whey protein hydrolysate according to the present invention is used in an infant formula, the protein content in the infant formula is in the range of 1.6 to 5.0 g/100 kcal. Besides from whey protein hydrolysate, the infant formula may also comprise carbohydrates, such as lactose, oligosaccharides, lipids, vitamins and minerals.

The whey protein hydrolysate according to the present invention may also be used in the preparation of other infant nutrition than infant formulas, for example in smoothies, porridge and the like.

The whey protein hydrolysate of the invention may also be used in the preparation of an emulsion. An emulsion will typically be prepared by reconstituting a powder of the whey protein hydrolysate in a liquid, e.g. water or milk, and fats. The whey protein hydrolysate will have an emulsifying effect on water and fats. The powder will typically comprise the whey protein hydrolysate in an amount of corresponding to from 5 to 15% by weight protein. After reconstitution, the emulsion comprises protein in an amount of from 2 to 4% by weight.

Hydrolysis of proteins causes changes of the protein, such as an increase in the number of charged groups, a decrease in the average molecular weight, and exposure of reactive groups. This is factors that influence emulsion-forming and emulsion-stabilizing abilities of protein hydrolysates. The whey protein hydrolysates of the present invention may be used as emulsifying agents, stabilizers, etc. by combining them with other ingredients.

The whey protein hydrolysates of the invention may also be used in bakery products, for example in bisquits, cookies and crackers.

In further aspects, the invention relates to the use of the whey protein hydrolysate according to the invention as an antioxidant. The inventors of the present invention have surprisingly found out that the whey protein hydrolysate of the invention have an antioxidative effect. Hence, the whey protein hydrolysate can be used in nutritional compositions as a source of antioxidative peptides.

Hence, the present invention relates to a whey protein hydrolysate of the invention having antioxidative effect. More particularly, the present invention relates to a whey protein hydrolysate having an antioxidative effect defined by a scavenging percentage from 54 to 60 in a solution comprising 1.5% by weight protein. The scavenging percentage is measured by the DPPH (2,2-diphenyl-1-picryl-hydrazyl-hydrate) assay. The scavenging percentage is calculated as $100_\times(A_0-A_S)/A_0$, where $A_0$ is the absorption in the absence of sample, and As is the absorbance in the presence of sample.

The invention will now be described in further details in the following non-limiting examples.

## Examples

### Example 1: Methods of analysis

Example 1.1: Determination of degree of hydrolysis (DH)

The degree of hydrolysis (DH) is defined as the percentage of peptide bonds cleaved by hydrolysis, see equation (1) below. The DH value gives information about the number of peptides formed which is related to the number of available peptide bonds.

The DH of the whey protein hydrolysates was measured as described in Adler-Nissen, J. Determination of the degree of hydrolysis of food protein hydrolysates by trinitrobenzenesulfonic acid. J. Agric. Food Chem. 27, 1256-1262 (1979). and Nielsen, P. M., Petersen, D. & Dambmann, C. Improved method for determining food protein degree of hydrolysis. J. Food Sci. 66, 642-646 (2001*).

In equation (1), h represents the number of cleaved peptide bonds and $h_{total}$ represents the total number of peptide bonds available. Thus, DH gives the percentage of cleaved peptide bonds.

$$\text{Equation (1):} \quad DH = (number\ of\ free\ amino\ terminals)/(total\ number\ of\ available\ peptide\ bonds)\cdot 100\ \% = h/h_{total}\ \cdot 100\ \%$$

The free alpha-amino groups formed after hydrolysis react with o-phthalaldehyde (OPA) and form a yellow complex which absorbs light at 340 nm and therefore can be measured spectrophotometrically. Based on the color formation, DH can be calculated.

Hydrolysates were resuspended in water at appropriate concentrations (0.03-0.08% protein) and 2 volumes were reacted for 2 minutes at $25^0$C with 15 volumes of OPA reagent (100 mM $Na_2B_4O_7$, 0.1% sodiumdodecyl-sulphate, 6 mM DL-dithioreitol, 6 mM o-phtalaldehyde and 2% ethyl alcohol). Similar reactions were made to create concentration series of L-serine. Next, the absorbance at 340 nm (A340) was measured and the A340 signal from the reaction of OPA with water was substracted. To find the true DH, the serine equivalents measured in the supernatants were corrected as suggested by Adler-Nissen for the Trinitrobenzenesulfonic acid method [Adler-Nissen J; Agricultural and Food Chemistry, 1979 27 (6) 1256] which give the same response as the described OPA method. The factors used for the whey protein hydrolysates were a=1, b=0.4, $h_{total}$=8.8.

Example 1.2: Determination of turbidity

Nephelometric turbidity of whey protein hydrolysates are used as a measurement for clarity and tranparency. If the nephelometric turbidity measured is lower than 100 NTU in a 4% w/w protein solution, the sample is perceived as transparent. Further, if the nephelometric turbidity measured is lower than 40 NTU in a 4% w/w protein solution, the sample is perceived as clear.

When measuring the nephelometric turbidity, a sample is diluted to 5 different concentrations of protein, 1.8%, 3.2%, 4.8%, 6.4% and 8% and the nephelometric turbidity is measured with a Turbiquant 3000 IR from Merck.

Example 1.3: Determination of total protein

The total protein content (protein equivalents) of a sample is determined by:

1) Determining the total nitrogen of the sample following ISO 8968-1/2IIDF 020-1/2-Milk - Determination of nitrogen content - Part 172: Determination of nitrogen content using the Kjeldahl method.
2) Calculating the total amount of protein as: Nx6.38

Example 1.4: Determination of total amino acid content

Analysis of the amino acid composition gives detailed information about the protein hydrolysate as a source of amino acid supplementation.

The total amino acid content was measured by the method of ISO 13903:2005, EU 152/2009. A sample was hydrolyzed in aqueous hydrochloric acid to break peptide bonds in the sample. After hydrolysis, the sample was pH adjusted, brought to volume and filtered. Amino acids were separated in an amino acid analyser and the detection was carried out using post column derivatisation using ninhydrin reagent and measured at 440 and 570 nm. For quantification, a 1-point calibration was used. For quality assurance, an in-house standard was analysed in every run.

Cyst(e)ine and methionine must be oxidised before analysis on the amino acid analyser. Samples were oxidized with hydrogen peroxide and formic acid at cold temperature, followed by acid hydrolysis using aqueous hydrochloric acid. The oxidation process oxidizes the methionine and cysteine, preventing loss during hydrolysis. After hydrolysis, the sample was analysed as described above. Quantification of total tryptophan: Tryptophan was analysed using another method, because tryptophan cannot be quantified in the same way as the other amino acids, as it is destroyed during acid hydrolysis of proteins. Instead, the sample was hydrolyzed by alkaline treatment and quantified by HPLC analysis. Results for each of the amino acids are normalized to the total amount of amino acids found: [g/100 g amino acids].

Example 1.5: Determination of free amino acid content

Free amino acids in the whey protein hydrolysates are determined by the methods of R. Schuster, "Determination of Amino Acids in Biological, Pharmaceutical, Plant and Food Samples by Automated Precolumn Derivatization and HPLC", Journal of Chromatography, 431:271-284 (1988) and Henderson, J.W., Ricker, R.D. Bidlingmeyer, B.A., Woodward, C., "Rapid, Accurate, Sensitive, and Reproducible HPLC Analysis of Amino Acids, Amino Acid Analysis Us-ing Zorbax Eclipse-AAA columns and the Agilent 1100 HPLC," Agilent Publication, 2000.

Free amino acids is determined by extracting amino acids into an aqueous or acidic solution. Samples may be deproteinated by molecular weight filtration. The samples are analysed by HPLC after pre-injection derivatisation. The primary amino acids are derivatised with o-phthalaldehyde and the secondary amino acids are derivatised with fluorenylmethyl chlorofomate before injection. The results are given as:

[mg free amino acid/100 g whey protein hydrolysate powder]

Example 1.6: Method to determine the peptide distribution in the whey protein hydrolysates

Size exclusion chromatography (SEC) was used to analyze the molecular weight distribution of the peptides in the whey protein hydrolysate. SEC is used to separate polymer type molecules by size. A mixture of components of different size, here peptides, can be separated by SEC. The elution time is dependent on the size of the molecule. The smaller the molecule the longer the elution time.

The samples were dissolved in the mobile phase to a concentration of 0.5 % w/v. Before injection the sample was filtered through a 0.45 $\mu$m filter. Chromatographic separation was performed on three TSK G2000 SWXL (125 Å, 5 $\mu$m, 7.5 mm $\times$ 300 mm) columns bound in series. A buffer of 0.0375 M phosphate buffer, 0.375 M ammonium chloride, 0.1 % Trifluoroacetic acid (TFA), and 25 % acetonitrile ($CH_3CN$) was used as the mobile phase with a flow of 0.7 mL per minute. Detection of the peptides were performed using a UV-detector measuring at 214 nm.

Based on the retention time, the distribution of peptides is divided according to size, and the relative amount is given according to the molecular weight.

**Example 2: Screening of enzyme combinations**

A total of 55 different enzyme combinations were tested for use in enzymatic hydrolysis of whey proteins. The obtained whey protein hydrolysates were analyzed with regard to clarity, bitterness, degree of hydrolysis, and peptide composition. The hydrolysis trials were made in a 0.5 liter scale.

The enzymes used for testing different enzyme combinations were serine endopeptidases from *Bacillus* (EC 3.4.21.62), serine endopeptidases from *Aspergillus* (EC 3.4.21), trypsin-like protease of microbial origin (EC 3.4.21.4), aminopeptidase from *Aspergillus* (EC 3.4.11), metalloendopeptidase from *Bacillus amyloliquefaciens* (EC 3.4.24.28),endoprotease from *Ananas comosus* (Bromelain) (EC 3.4.22.32), a proline specific endopeptidase from *Aspergillus niger* (EC: 3.4.21.26), an aminopeptidase preparation from *Aspergillus oryzae* (EC 3.4.11, a metalloendopeptidase preparation from *Geobacillus stearothermophilus* (EC 3.4.24) and an endopeptidase preparation from *Bacillus amyloliquefaciens* (EC 3.4).

The enzyme preparations used for the experiment were:

Protamex (Novozymes A/S) which comprises a serine endopeptidase from *Bacillus* sp. (subtilisin) and bacillolysin. Protease A Amano 2 SD (Amano Enzymes Ltd.), comprising serine endopeptidase from *Apergillus oryzae*

Promod 782 MDP (Biocatalysts Ltd.), which comprises serine endopeptidases from *Aspergillus sp.*

Formea TL 1200 BG (Novozymes A/S), comprising trypsin-like protease of microbial origin

Promod 950L (Biocatalysts Ltd.), comprising serine endopeptidases from *Bacillus* sp. (subtilisin) and bacillolysin.

Flavourzyme conc BG (Novozymes A/S), comprising leucyl aminopeptidases from *Aspergillus oryzae*

Alcalase AF 2.4 L (Novozymes A/S), comprising serine endopeptidases (subtilisin) from *Bacillus licheniformis*

Neutrase conc BG (Novozymes A/S), comprising metalloendopeptidases (bacillolysin) from *Bacillus amyloliquefaciens*

Promod 523 MDP (Bromelain) (Biocatalysts Ltd.), comprising cysteine endopeptidases from *Ananas comosus*

Maxipro PSP (DSM), proline specific endopeptidase from *Aspergillus niger* Flavorpro 766 (Biocatalyst Ltd.), comprising aminopeptidases from *Aspergillus oryzae*

Thermoase PC10F (Amano Enzymes Ltd.), comprising metalloendopeptidase from *Geobacillus stearothermophilus*

Protin NY100 (Amano Enzymes Ltd.), comprising endopeptidases from *Bacillus amyloliquefaciens*

55 combinations of enzymes were used in hydrolysis of whey protein. As substrate for the hydrolysis, a solution of a whey protein isolate (WPI) (Lacprodan DI-9224 from Arla Food Ingredients) was used. The solution of WPI for all experiments had a protein concentration of 8% by weight. The hydrolysis reaction was performed at 50°C, with pH-stat at pH=7 and a reaction time of 6 hours after the first enzyme was added. After 6 hours of hydrolysis, the hydrolysis was stopped by heating to 99°C with a holding time of 90 seconds to inactivate the enzymes. The amount of the enzymes used is mentioned in Table 1 as the amount of enzyme in gram per 100 gram protein. Product from each hydrolysis trial was freeze-dried and used for further analyses including clarity (in form of turbidity measurements), degree of hydrolysis and taste evaluation and scoring. The results are given in Table 1 below:

NEU: refers to Neutrase
FZ: refers to Flavourzyme conc BG
Alca: refers to Alcalase AF 2.4 L
PA: refers to Protease A Amano 2 SD
FTL: refers to Formea TL 1200 BG
PM782: refers to Promod 782 MDP
FP766: refers to Flavourpro 766
MP PSP: refers to Maxipro PSP
THER: refers to Thermoase PC10F
PRO: refers to Protin NY100
PM950: refers to Promod 950L
PTM: refers to Protamex

The enzymes used for the trials presented in Table 1 were selected among a larger number of enzymes based on trials in 96-well scale. These trials included hydrolysis with different enzyme combinations and visual scoring of apparent clarity and heat stability and hydrolysis (SDS-PAGE). To allow pH-stat hydrolysis and larger amounts of material for analysis the combinations presented in Table 1 were done in 500 ml scale as described above. The enzymes presented in Table 1 were therefore not randomly selected. Some of the enzyme combinations in Table 1 were repeated at different doses (e.g. combination 1-3).

The term "Visual a inact." In table 1 refers to "visual after inactivation

Table 1

| Sample ID | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| Enz 1 (dose) | PTM 0.156 | PTM 0.312 | PTM 0.625 | PTM 0.625 | PA 0.312 | PA 0.312 | 0.312 PA | PTM 0.312 | PTM 0.312 | PM 950 3.125 | Alca 1.05 | PA 0.312 | PA 0.312 | THER 0.312 |
| Enz 2 (dose) | PA 0.156 | PA 0.312 | PA 0.625 | PM782 0.625 | | | | PA 0.312 | PA 0.312 | PA 0.312 | PA 0.312 | FTL 0.312 | FTL 0.312 | PA 0.312 |
| Enz 3 (dose) | FTL 0.156 | FTL 0.312 | FTL 0.625 | FTL 0.625 | FTL 0.312 | FZ 0.312 | FP766 0.312 | FZ 0.312 | FZ 0.312 | FZ 0.312 | FZ 0.312 | FZ 0.312 | FP766 0.312 | FZ 0.312 |
| Visual a inact. | clear | clear | clear | clear | clear | turbid | turbid | turbid | turbid | clear | clear | clear | clear | turbid |
| DH-% | | | | 22.1 | | | | | | 31.5 | 31.5 | | | |
| Bitter taste | 4% | 4% | 4% | >4% | 4% | 4% | 4% | 8% | 8% | 8% | 8% | 4% | 4% | 8% |
| >2500 Da (5%) | 36.4 | 28.6 | 20.9 | 17.6 | 34.5 | 27.7 | 41 | 21.1 | 33.7 | 11.7 | 9.6 | 17.8 | 21.2 | 20.7 |

EP 3 975 733 B1

| Sample ID | 15 | 16 | 17 | 18 | 19 | 20 | 21 | 22 | 23 | 24 | 25 | 26 | 27 | 28 |
|---|---|---|---|---|---|---|---|---|---|---|---|---|---|---|
| Enz 1 (dose) | PRO 0.312 | PM782 0.625 | PM782 0.625 | PM782 0.625 | PM782 0.625 | PM782 0.625 | PM782 0.625 | PM782 0.625 | Neu 0.219 | Neu 0.219 | Neu 0.219 | Neu 0.219 | Neu 0.219 | Neu 0.219 |
| Enz 2 (dose) | PA 0.312 | FTL 0.625 | FTL 0.624 | FTL 0.625 | FTL 0.625 | FTL 0.625 | FTL 0.625 | FTL 0.625 | PM523 0.625 | Alca 6,25 | PM950 6.25 | PM523 0.625 | PM523 0.625 | PM523 0.625 |
| Enz 3 (dose) | FZ 0.312 | FZ 0.625 | FP766 0.625 | FZ 0.625 | FP 766 0.625 | FZ 0.625 | FZ 0.625 | FZ 0.625 | PM782 0.625 | PM782 0.625 | PM782 0.625 | MPPSP 6.25 | MPCPP 6.25 | |
| Visual a inact. | turbid | gel | gel | clear | turbid | clear | turbid | white | clear | turbid | turbid | clear | gel | clear |
| DH-% | | 22.6 | 16.6 | 34.7 | 18.8 | 52.4 | 55.4 | 54.5 | 18.7 | 24.5 | 21.7 | 14.7 | 16.3 | 15.3 |
| Bitter taste | 8% | N/A | N/A | 4% | 8% | 4% | 4% | 8% | 4% | 2% | 4% | 4% | 4% | 8% |
| >2500 Da (5%) | 31.9 | 36.0 | 40.5 | 15.8 | 27.6 | 19.2 | 16.5 | 17.7 | 26.9 | 5.4 | 14.6 | 26.8 | 20.8 | 24.6 |

| Sample ID | 29 | 30 | 31 | 32 | 33 | 34 | 35 | 36 | 37 | 38 | 39 | 40 | 41 | 42 |
|---|---|---|---|---|---|---|---|---|---|---|---|---|---|---|
| Enz 1 (dose) | Neu 0.219 | Neu 0.219 | Neu 0.129 | Neu 0.219 | Neu 0.219 | PM523 0.625 | PM523 0.625 | PM523 0.625 | PM523 0.625 | PTM 0.625 | PTM 0.625 | PTM 0.625 | PM523 0.625 | Neu 0.219 |
| Enz 2 (dose) | PM782 0.625 | PM523 0.625 | PM523 0.625 | MPPSP 6.25 | PM523 0.625 | PM144 0.625 | Alca 6.25 | PM950 6.25 | PTM 0.625 | PM523 0.625 | PM782 0.625 | MPPSP 6.25 | Neu 0.219 | PM523 0.625 |
| Enz 3 (dose) | FZ 0.125 | FZ 0.125 | FTL .0625 | PM782 0.625 | FP766 0.625 | PM782 0.625 | FP782 0.625 | PM782 0.625 | PM782 0.625 | Neu 0.219 | Neu 0.219 | Neu 0.219 | FZ .0625 | FZ .0625 |
| Visual a inact. | turbid | clear | turbid | clear | clear | clear | white | white | clear | turbid | turbid | clear | clear | clear |
| DH-% | 25.4 | 25.6 | 18.8 | 20.5 | 19.6 | 15.8 | 24.4 | 22.5 | 18.8 | 17.9 | 20.8 | 14.5 | 22.8 | 22.4 |
| Bitter taste | 8% | 8% | 8% | 8% | 8% | 8% | 2% | 4% | 8% | 4% | 4% | 2% | N/A | N/A |
| >2500 Da (5%) | 26.7 | 15.3 | 14.1 | 15.8 | 17.8 | 20.8 | 3.5 | 8.5 | 24.3 | 17.3 | 27.3 | 48.9 | 19.3 | 35.2 |

| Sample ID | 43 | 44 | 45 | 46 | 47 | 48 | 49 | 50 | 51 | 52 | 53 | 54 | 55 |
|---|---|---|---|---|---|---|---|---|---|---|---|---|---|
| Enz 1 (dose) | Neu 0.219 | Neu 0.219 | PTM 0.625 | PTM 0.625 | PM523 0.625 | Neu 0.219 | Neu 0.219 | Neu 0.219 | PTM 0.625 | Neu 0.219 | Neu 0.219 | Neu 0.219 | PTM 0.625 |
| Enz 2 (dose) | PTM 0.625 | MPPSP | Neu 0.219 | Neu 0.219 | Neu 0.219 | PM782 0.625 | PTM 0.625 | MPPSP 6.25 | Neu 0.219 | PM523 0.625 | PM782 0.625 | MPPSP 6.25 | Neu 0.219 |
| Enz 3 (dose) | FZ .0625 | FZ .0625 | FZ .0625 | PM782 0.625 | FP766 0.625 | FP766 0.625 | FP766 0.625 | FP766 0.625 | FP766 0.625 | PTM 0.625 | PTM 0.625 | PTM 0.625 | MPPSP 6.25 |
| Visual a inact. | clear | tubid | turbid | turbid | clear | turbid | clear | clear | clear | turbid | turbid | clear | clear |
| DH-% | 19.9 | 17.7 | 17.9 | 8.1 | 8.1 | 8.2 | 8.1 | 16.3 | 17.3 | 18.3 | 23.6 | 14.9 | 15.5 |
| Bitter taste | 4% | 4% | 4% | 4% | 8% | 8% | 4% | 8% | 4% | 8% | 6% | 8% | 8% |
| >2500 Da (5%) | 28.7 | 42.6 | 34.3 | 28.7 | 22.1 | 26.6 | 33.6 | 45.3 | 37.0 | 15.3 | 26.7 | 43.4 | 39.7 |

The data were evaluated for each whey protein hydrolysate. For a positive evaluation, the following conditions should be satisfied:

- clear in appearance after enzyme inactivation
- having a high degree of hydrolysis above 15%, and preferably above 20%
- have no bitter taste in a 4% w/w protein solution
- 25% by weight or less of the peptides should have a molecular weight above 2500 Da

The samples should be clear after 90 seconds at 99°C as this is indicative of UHT stability (stable and clear after treatment of a 4% solution at 143°C for 6 seconds.

Hence, it can be seen from the above tables that 4 out of the 55 enzyme combinations, i.e. the samples referred to as sample 4, 10, 11 and 30, fulfilled the conditions.

Sample 4 was obtained by hydrolysis with a combination of the enzymes serine endopeptidase from *Bacillus,* serine endopeptidase from *Aspergillus,* and trypsin-like protease

Sample 10 was obtained by hydrolysis with a combination of the the enzymes serine endopeptidase from *Bacillus,* metallo endopeptidase from *Bacillus,* serine endopeptidase from *Aspergillus,* and leucyl aminopeptidase from *Aspergillus.* Sample 11 was obtained by hydrolysis with serine endopeptidase from *Bacillus,* serine endopeptidase from *Aspergillus,* and leucyl aminopeoptidase from *Aspergillus.*

Sample 30 was obtained by hydrolysis with bacillolysin from *Bacillus amyloliquefaciens,* bromelain and leucyl aminopeoptidase from *Aspergillus.*

**Example 3: Further analysis of whey protein hydrolysates according to the invention.**

The four whey protein hydrolysates from example 2 that fulfilled the conditions of clarity, taste, degree of hydrolysis and peptide distribution were analyzed further and mentioned as sample 1-4 (S1-S4) in the table below.

Sample 5-12 (S5-S12) are whey protein hydrolysates from hydrolysis with other enzyme combinations.

Sample 13 (S13) is a spray-dried WPI hydrolysate obtained by hydrolysis of sweet whey with a combination of subtilisin from *Bacillus licheniformis* (Alcalase) and bacillolysin from *Bacillus amyloliquefaciens* (Neutrase), followed by ultrafiltraton, treatment with activated charcoal and microfiltration to remove the activated charcoal (as described in WO 1993/024020A1). The degree of hydrolysis is about 25%.

Sample 14 (S14) is prepared by using the same enzyme combination as sample 4 (enzyme combination of the invention) but where a WPC is used as substrate for the hydrolysis.

Sample 15 and 16 (S15-S16) are replicates of sample 4. Sample 15 and 16 are WPI based hydrolysates prepared without UF filtration using the same conditions as sample 4.

The whey protein hydrolysates made by enzymatic hydrolysis with different enzyme combinations were again analyzed for being clear, the degree of hydrolysis and the content of peptides having a molecular weight of above 2500 Da and the content of peptides having a molecular weight below 375 Da.

The clarity (turbidity) of samples was determined by measurements according to example 1.2 and the nephelometric turbidity should be lower than 40 NTU for the sample to be perceived as clear and below 100 NTU for the sample to be perceived as transparent.

A sample was scored as being bitter if bitter taste was perceived at 4% protein or less.

The degree of hydrolysis was measured by the method described in example 1.1 while the bitterness was measured by tasting the sample in different concentrations (2%, 4%, 8% protein concentration). The distribution of peptides was measured as described in example 1.5. The result is shown in table 2:

Table 2:

| | Origin | Enzyme combination | Clear (nephelometric turbidity at 4% w/w protein lower than 40 NTU) | Bitter at % protein | DH (%) | >2500 Da (%) | <375 Da (%) |
|---|---|---|---|---|---|---|---|
| S1 | WPI | Serine endopeptidase from *Bacillus* Serine endopeptidase from *Aspergillus* Leucyl aminopeptidase | Yes | 8 | 31.5 | 11.7 | 20.4 |

(continued)

| | Origin | Enzyme combination | Clear (nephelometric turbidity at 4% w/w protein lower than 40 NTU) | Bitter at % protein | DH (%) | >2500 Da (%) | <375 Da (%) |
|---|---|---|---|---|---|---|---|
| S2 | WPI | Serine endopeptidase from *Bacillus* Serine endopeptidase from *Aspergillus* Trypsin-like protease | Yes | >4 | 31.5 | 17.6 | 13.5 |
| S3 | WPI | Serine endopeptidase from *Bacillus* Serine endopeptidase from *Aspergillus* Leucyl aminopeptidase | Yes | 8 | 22.1 | 9.6 | 22.2 |
| S4 | WPI | Bacillolysin from *Bacillus amyloliquefaciens* Bromelain Leucyl aminopeptidase | Yes | 8 | 25.6 | 15.3 | 14.4 |
| S5 | WPI | Serine endopeptidase from *Aspergillus* Trypsin-like protease Leucyl aminopeptidase | No | 8 | 18.9 | 27.6 | 8.7 |
| S6 | WPI | Bacillolysin from *Bacillus amyloliquefaciens* Serine endopeptidase from *Bacillus* Serine endopeptidase from *Aspergillus* | No | 2 | 24.6 | 5.4 | 21 |
| S7 | WPI | Bromelain Serine endopeptidase from *Bacillus* Metallo endopeptidase from *Bacillus* Serine endopeptidase from *Aspergillus* | Yes | 8 | 18.9 | 24.5 | 9.9 |
| S8 | WPI | Bacillolysin from *Bacillus amyloliquefaciens* Serine endopeptidase from *Bacillus* Serine endopeptidase from *Aspergillus* | No | 4 | 21.7 | 14.6 | 13.8 |
| S9 | WPI | Bacillolysin from *Bacillus amyloliquefaciens* Serine endopeptidase from *Aspergillus* Leucyl aminopeptidase | No | 8 | 23.4 | 26.7 | 13.8 |
| S10 | WPI | Bacillolysin from *Bacillus amyloliquefaciens* Prolyl specific endopeptidase Serine endopeptidase from *Aspergillus* | Yes | 8 | 20.5 | 28.4 | 24.3 |
| S11 | WPI | Bromelain Serine endopeptidase from *Bacillus* Serine endopeptidase from *Aspergillus* | No | 2 | 24.4 | 1.9 | 54.5 |
| S12 | WPI | Bromelain Serine endopeptidase from *Bacillus* Serine endopeptidase from *Aspergillus* | No | 4 | 22.5 | 8.5 | 16.6 |
| S13 | WPC | Serine endopeptidase from *Bacillus* Bacillolysin from *Bacillus amyloliquefaciens* | No | >2 | 23.0 | 7.5 | 18.9 |
| S14 | WPC | Bacillolysin from *Bacillus amyloliquefaciens* Bromelain | No | 8 | 25.4 | 23.2 | 15 |

(continued)

|  | Origin | Enzyme combination | Clear (nephelometric turbidity at 4% w/w protein lower than 40 NTU) | Bitter at % protein | DH (%) | >2500 Da (%) | <375 Da (%) |
|---|---|---|---|---|---|---|---|
|  |  | Leucyl aminopeptidase |  |  |  |  |  |
| S15 | WPI | Bacillolysin from *Bacillus amyloliquefaciens* Bromelain Leucyl aminopeptidase | Yes | 8 | 23.0 | 23.2 | 12.7 |
| S16 | WPI | Bacillolysin from *Bacillus amyloliquefaciens* Bromelain Leucyl aminopeptidase | Yes | 8 | 23.0 | 17.9 | 13.5 |

Hence, from table 2, it is shown that the use of specific enzyme combinations according to the present invention results in whey protein hydrolysates that 1) have a degree of hydrolysis above 20%, 2) 8 to 25% of the peptides have a molecular weight of 2500 Da or above and 3) have no bitter taste at a concentration of 4% protein or below.

In addition, table 2 shows that if a WPI is used for the protein hydrolysis, the use of the specific enzyme combinations of the invention results in whey protein hydrolysates that are clear in appearance. Table 2 also shows that the use of the specific enzyme combinations in the preparation of whey protein hydrolysates where a WPC is used as the substrate results in a whey protein hydrolysate having a degree of hydrolysis above 20%, 8 to 25% of the peptides have a molecular weight of 2500 Da or above and have no bitter taste at a concentration of 4% protein or below. However, the whey protein hydrolysates prepared by using a WPC as a substrate is not clear in appearance (because of the lipids present in WPC).

In a preferred embodiment of the invention, a WPI is used as the substrate for protein hydrolysis to obtain clear hydrolysates.

### Example 4: Turbidity analysis

Nephelometric turbidity at different protein concentrations of sample 1 to 16 from example 3 was further analyzed. Samples with a turbidity of less than 100 NTU were considered transparent and samples with a turbidity below 40 was considered clear. Table 3 below shows the turbidities of the hydrolysates mentioned as sample 1-12 in example 3 measured at different protein concentrations. The protein concentrations are 1.8% protein, 3.2% protein, 4.8% protein, 6.4% protein and 8% protein. The protein concentration is percentage by weight.

Powders of the whey protein hydrolysates were hydrated for at least 30 minutes at the indicated concentrations before the turbidities were measured (n=3).

Table 3: Turbidities (NTU) of trial samples at 1.8. 3.2. 4.8. 6.4 and 8% protein

| Protein content (%-wt) | 1.8 | 3.2 | 4.8 | 6.4 | 8 |
|---|---|---|---|---|---|
| Sample 1 | 9.26 | 18.69 | 29.05 | 34.14 | 43.48 |
| Sample 2 | 14.46 | 26.86 | 34.38 | 42.45 | 50.15 |
| Sample 3 | 10.93 | 21.08 | 30.48 | 41.29 | 48.56 |
| Sample 4 | 11.58 | 23.78 | 33.82 | 43.73 | 63.41 |
| Sample 5 | 129.42 | 213.08 | 269.67 | 292.63 | 305.15 |
| Sample 6 | 177.24 | 366.35 | 620.99 | 827.23 | 921.99 |
| Sample 7 | 12.4 | 21.5 | 31.31 | 37.11 | 42.04 |
| Sample 8 | 86.94 | 186.66 | 254.45 | 336.05 | 399.52 |
| Sample 9 | 68.41 | 121.46 | 182.88 | 236.2 | 263.59 |
| Sample 10 | 16.5 | 31.52 | 46.38 | 58.8 | 67.4 |
| Sample 11 | 154.82 | 318.38 | 511.59 | 740.01 | 972.62 |

(continued)

| Protein content (%-wt) | 1.8 | 3.2 | 4.8 | 6.4 | 8 |
|---|---|---|---|---|---|
| Sample 12 | 114.53 | 228.26 | 334.15 | 435.53 | 575.48 |

Hence, it is shown from table 3 that the specific enzyme combinations according to the invention has a turbidity of less than 100 NTU at concentrations of 8% protein.

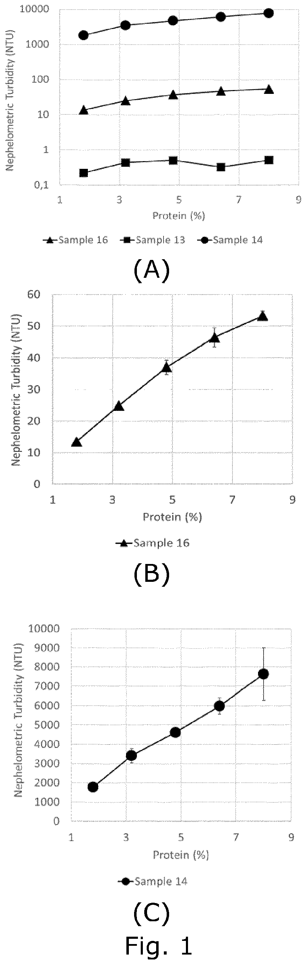

In figure 1 A-C, the measured turbidities of sample 13, 14 and 16 are shown. Figure 1 A shows the turbidity of the whey protein hydrolysates of sample 13, 14 and 16 and shows the difference in turbidity. From figure 1A, it is shown that a whey protein hydrolysate (not the invention, sample 13) subjected to ultrafiltration has a very low turbidity (below 1 NTU) and is the hydrolysate being most clear. The hydrolysate prepared according to the method according to the present invention (sample 16) using one of the specific enzyme combinations has a turbidity below 100 NTU at a 8% protein concentration and is therefore transparent. Furthermore, the hydrolysate of the invention has a turbidity below 40 NTU at a 4% protein concentration and is therefore perceived as clear in a 4% protein concentration. On the contrary, the whey protein hydrolysate prepared according to the invention by using a WPC as substrate (sample 14) has a turbidity above 1000 NTU and is therefore perceived as unclear.

Figure 1B, more clearly shows that the turbidity of sample 16 is less than 40 NTU at a protein concentration of 5% or less.

Figure 1C shows the turbidity of sample 14. It is shown that even at very low concentrations (about 2%), the turbidity is above 2000 NTU. Sample 14 is perceived as very unclear and not transparent.

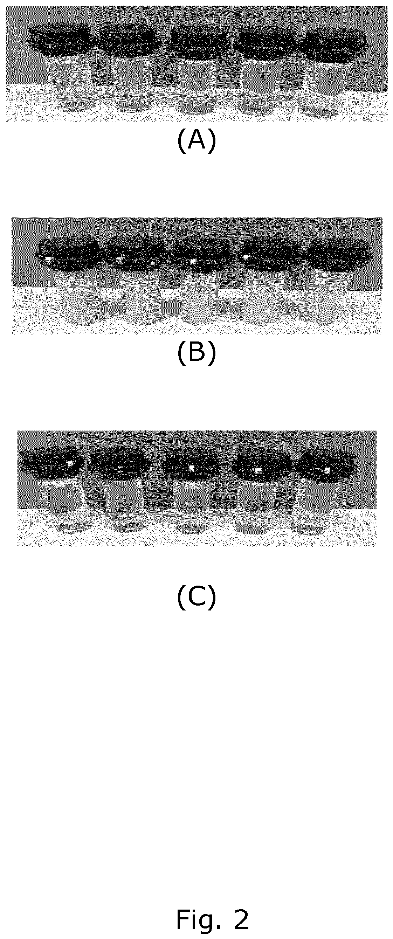

Figure 2 A-C include pictures of the sample 13, 14 and 16 to visually show clear samples versus unclear samples. The samples from left to right were prepared at 8%, 6.4%, 4.8%, 3.2% and 1.8% protein.

Figure 2A shows that sample 16 is visually clear and transparent at 8%, 6.4%, 4.8%, 3.2% and 1.8% protein.

Figure 2B shows sample 14, and it is shown that sample 14 is unclear and not transparent even in the lowest protein concentration of 1.8%.

Figure 2C shows sample 13, and it is shown that sample 13 is visually clear and transparent at all concentrations.

### Example 5: Bitterness evaluation in relation to caffeine

In Example 5 the bitterness of a whey protein hydrolysate according to the present invention is being analyzed as compared to a UF filtered and activated charcoal treated whey protein hydrolysate made by enzymatic hydrolysis of WPI with other enzymes than in the present invention (hydrolysis with a subtilisin from *Bacillus licheniformis* (Alcalase) and a bacillolysin from *Bacillus amyloliquefaciens* (Neutrase)).

A sensory evaluation was made to compare the taste of samples 13, 15 and 16 of example 3.

A sensory panel was trained to detect and quantify bitterness using a caffeine solution as reference. The training included that the panelists were first served reference samples including solutions composed of increasing concentrations of caffeine. The panelists were trained to assign bitterness scores to unknown solutions based on a 15 cm scale. The reference samples consisted of 3 caffeine solutions containing 0.025%, 0.05% and 0.1% caffeine respectively. After evaluation of the reference samples, the panelists tasted the hydrolysate samples 3 times in a 4% by weight protein solution in a randomized order and ranked the bitter intensity of the test solutions based on the bitterness in the reference solutions. The reference solution comprising 0.025% caffeine was perceived as not being bitter and the reference solution comprising 0.1% caffeine was perceived as bitter (scored 13 on a 15 cm scale of bitterness). The sensory panel contained 7 panelists participating in the evaluation.

In addition, a sensory profiling was carried out according to the international standard Quantitative Descriptive Profile ISO 13299:2016 2nd ed. 5.5, Annex F1-F6&H3 with use of the panel. 7 trained assessors participated in the evaluation. The evaluation was done in 3 repetitions. The response scale used was a continuous line scale (15 cm). Samples of approximately 2 ml were served at ambient temperature. Red light was used during the evaluation.

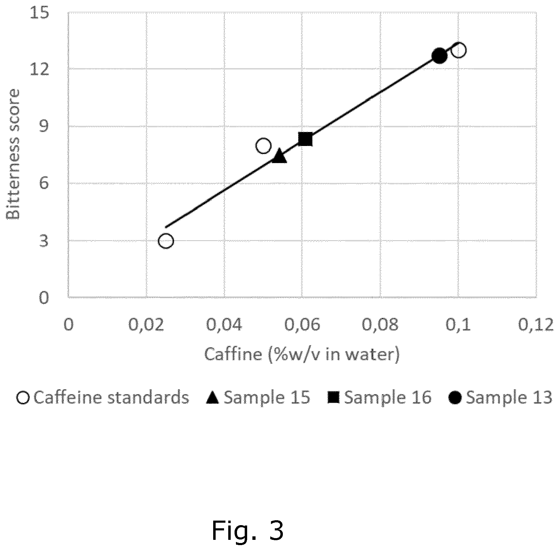

The bitterness scored by the sensory panel with reference to the bitterness of caffeine is shown in figure 3 where the bitterness score of the test samples is plotted against the concentration of caffeine. The bitterness of the 3 different concentrations of caffeine and the bitterness scores of sample 13, 15 and 16 from example 3 is shown in figure 3.

Figure 3 shows that sample 13 (hydrolysate of WPI but not with the enzyme combination of the invention, but the hydrolysate is ultrafiltered and treated by activated charcoal) has the highest relative bitterness of 0,095%. Sample 15 (hydrolysate of WPI using the enzyme combination of the invention, with no ultrafiltration and activated charcoal treatment) has the lowest relative bitterness out of the 3 product samples with a relative bitterness of 0,054%, but was quite close to the hydrolysate in sample 16 (similar to sample 15) which has a relative bitterness of 0,061% relative to caffeine.

Hence, the taste of the palatable whey protein hydrolysates prepared according to the invention (sample 15 and sample 16) was perceived as being less bitter than 0.08% of caffeine and less bitter than sample 13.

**Example 6: Taste profiling**

An example was made to evaluate the taste profile of the whey protein hydrolysates in samples 13, 15 and 16. The taste profiling was made by a trained panel and 5 attributes were used to identify differences between the samples with focus on odor, mouthfeel and taste.

Data were analyzed to identify significant differences between samples for each attribute. A statistical evaluation of the data is shown in table 4. Furthermore, a multiple range test has been used to identify the differences between the samples. In table 4, samples with the same letter are not significantly different.

Table 4: Sensory scores

| | Cheese_O *** | Broth_O *** | Astringent_ MF*** | Umami_T *** | Bitter_T *** |
|---|---|---|---|---|---|
| Sample 15 | 5.33$^A$ | 4.89$^A$ | 5.79$^A$ | 7.15$^A$ | 7.53$^A$ |
| Sample 13 | 1.4$^B$ | 1.14$^B$ | 10.71$^B$ | 1.54$^B$ | 12.3$^B$ |
| Sample 16 | 4.1$^A$ | 7.58$^C$ | 5.96$^A$ | 8.49$^A$ | 8.11$^A$ |
| *** $p<0.001$ Duncan test: Samples with different letter for an attribute are significantly different at 95% level ($p<0.05$) | | | | | |

Hence, the bitterness score of samples 15 and 16 is less than the bitterness score of sample 13.

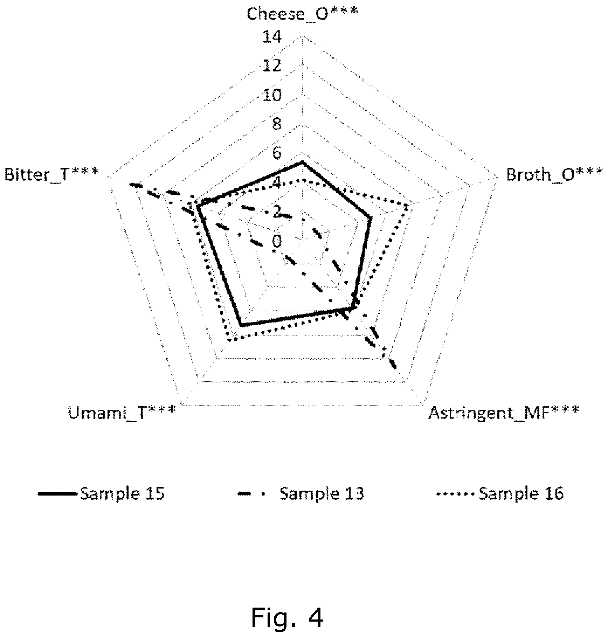

The data mentioned in table 4 can be represented as a spider web representation, see figure 4. The spider web shows the attributes of Odour (O), Mouthfeel (MF) and Taste (T). In the periphery of the plot 'high' intensity of each sensory attribute is shown and 'little' intensity is in the centre of the plot. Each product has a different label which is shown below the figure. As an example, see data for "Bitter_T" where *** indicates that data for sample 13 and samples 15 and 16 are significantly different ($p< 0.001$).

From the spider web representation of the taste profile, it is shown that sample 13 is very different from samples 15 and 16 in the taste profile and most importantly, samples 15 and 16 have a low bitterness.

**Example 7: Analysis by LC-MS/MS and peptide cleavage pattern**

Peptides in liquid samples can be identified by mass spectrometric peptide analysis and database searching. The samples 1, 2, 3, 13, 15 and 16 were analysed by LC-MS/MS to identify peptide sequences and the proteins they were derived from. The peptides present in the respective samples were dissolved in water and injected on a Dionex nano-LC system for MS/MS analysis on a Bruker Maxis Impact QTOF mass spectrometer. Searching with the acquired MS/MS spectra was done against a custom made database containing protein sequences of bovine origin. The overall results of the analyses are illustrated in table 5.

Table 5: Proteins identified by LC-MS/MS

| Coverage (%) | Sample 1 | Sample 2 | Sample 3 | Sample 13 | Sample 15 | Sample 16 |
|---|---|---|---|---|---|---|
| Beta-lactoglobulin | 79.8 | 80.9 | 82.6 | 74.2 | 82 | 82.6 |
| Beta-casein | 79.9 | 77.7 | 79.5 | 71.4 | 64.7 | 62.5 |
| Kappa-casein | 29.5 | 38.9 | 32.6 | 30.5 | 36.3 | 33.2 |
| GDCA molecule 1* | 46.4 | 65.4 | 34.6 | 48.4 | 56.2 | 40.5 |
| Osteopontin | 40.6 | 30.9 | 36.7 | 39.9 | 33.8 | 29.9 |
| Alpha-lactalbumin | 42.3 | 54.2 | 58.5 | 47.2 | 53.5 | 62 |
| Alpha-S1-casein | 49.1 | 53.7 | 41.1 | 36 | 41.1 | 36.4 |
| BSA** | 18.9 | 15 | 21.3 | 10.2 | 8.4 | 10.5 |
| Alpha-S2-casein | 27 | 33.3 | 20.7 | 23.9 | 19.4 | 11.3 |

(continued)

| Coverage (%) | Sample 1 | Sample 2 | Sample 3 | Sample 13 | Sample 15 | Sample 16 |
|---|---|---|---|---|---|---|
| Total No peptides identified | 776 | 917 | 709 | 631 | 595 | 615 |
| *Glycosylation-dependent cell adhesion molecule 1<br>** Bovine serum albumin | | | | | | |

The data in Table 5 showed that the analyses provided a high sequence coverage for the most prevalent proteins in the samples including beta-lactoglobulin, alpha-lactalbumin and beta-casein. Furthermore, the number of peptides identified from each sample is indicated. As the datasets were of the expected quality for LC-MS/MS analysis they were valid for more detailed analysis (example 9).

**Example 8: LS-MS/MS analysis of bitter peptides**

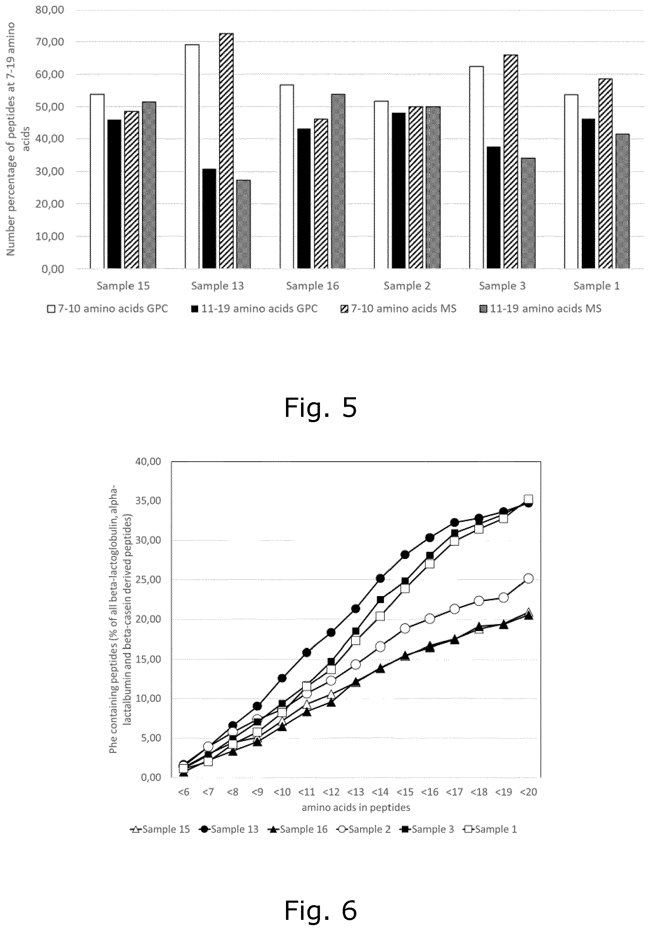

First it was confirmed that the LC-MS/MS data from example 7 corresponded to data obtained by SEC analysis (Size Exclusion Chromatography) by comparing the peptide distributions. Here the SEC data were converted to number of percentages (the number of peptides of a given molecular weight) and plotted for each hydrolysate together with the same data calculated based on LC-MS/MS (see figure 5). The LC-MS/MS data only included peptides from beta-lactoglobulin whereas the SEC analysis accounted for all protein in the samples.

From figure 5, it is shown that the percentage of beta-lactoglobulin derived peptides in the range of 7-10 amino acids and in the range of 11-19 amino acids of the number of beta-lactoglobulin derived peptides at 7-19 amino acids were similar when using LC-MS/MS and SEC.

Hence, the data shown in figure 5 shows that the number of peptides in different amino acid length ranges in the LC-MS/MS dataset may be used to extract quantitative information about the relative distribution of peptides as data were comparable to SEC data and the SEC method is quantitative. In addition, figure 5 shows that sample 13 (outside the invention) had more peptides with a smaller peptide size than the hydrolysates according to the invention.

The amount of detectable peptides comprising phenylalanine as a percentage of the total number of peptides were identified and analysed in the samples 1, 2, 3, 13, 15 and 16 by MS-LC/MS. The result is shown in figure 6.

Without being bound by any theory, the inventors of the present invention believes that the presence of phenylalanine in peptides is correlated to bitterness, and the bitterness of phenylalanine may be enhanced when its amino- or carboxy-terminal is blocked by a peptide bond to another amino acid residue. For example, the phenylalanine residue in the bitter peptide YPFPGPIPN identified in a bitter whey protein hydrolysate was suggested to be a primary bitterness determinant (Liu. X.. Jiang. D.. and Peterson. D.G. Identification of Bitter Peptides in whey protein hydrolysate. J. Agric. Food Chem. 2014. 62: 5719-5725).

Figure 6 shows that the phenylalanine content in peptides as a percentage of the total number of peptides originating from beta-lactoglobulin, alpha-lactalbumin and beta-casein. The percentage of peptides comprising phenylalanine was lower for the less bitter hydrolysates at peptide sizes of less than 9 amino acid residues. Hence, for the whey protein hydrolysate according to the invention in sample 1 and 3, phenylalanine is present in peptides with a larger peptide size. The whey protein hydrolysates according to the invention represented by sample 2, 15 and 16, also have a low percentage of phenylalanine comprising peptides but in addition, sample 2, 15 and 16 also exhibited a lower percentage of phenylalanine bound in peptides overall. This indicates that there may be more phenylalanine present as free amino acids in samples 2, 15 and 16 than in samples 1, 3 and 13. Hence, an indication of non-bitter whey protein hydrolysates according to the present invention is that a large percentage of phenylalanine is either precent in larger peptides or present as free amino acids.

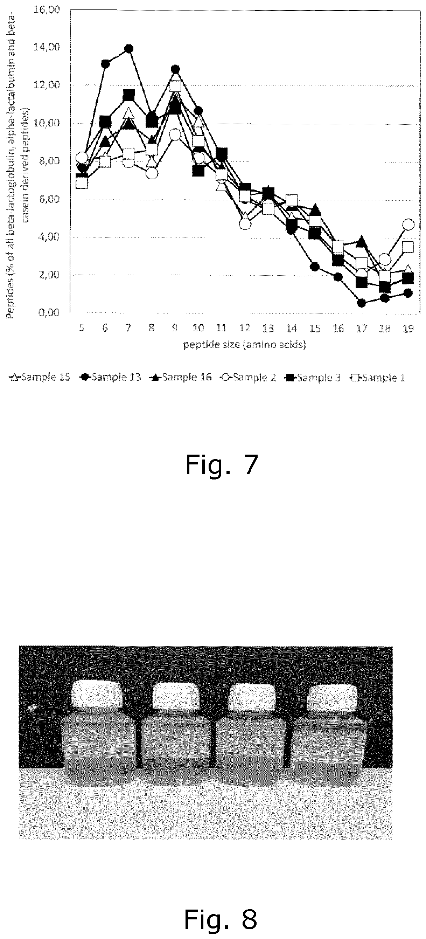

In figure 7, the percentage of peptides of 5-19 amino acids from beta-lactoglobulin, alpha-lactalbumin and beta-casein is shown.

From figure 7 it is shown that sample 13 (outside the invention) comprises more small peptides (5-9 amino acids) than the other five whey protein hydrolysates. Without being bound by any theory, the inventors of the present invention believes that the higher content of phenylalanine comprising peptides of smaller peptide sizes in the hydrolysate in sample 13 may be the reason for the higher bitterness in sample 13 than in samples 1, 2, 3, 15 and 16 which are hydrolysates according to the invention.

**Example 9: SEC size distribution data**

The size of the peptides in the samples 1, 2, 3, 13, 15 and 16 were analysed by Size Exclusion Chromatography (SEC). The results are shown in table 6 below:

Table 6: SEC size distribution data, DH and free amino acid (FAA) content for the whey protein hydrolysates in sample 1, 2, 3, 13, 15 and 16

| | Sample 1 | Sample 2 | Sample 3 | Sample 13 | Sample 15 | Sample 16 |
|---|---|---|---|---|---|---|
| <375 Da (%) | 20.4 | 13.5 | 22.2 | 16.1 | 13 | 13.5 |
| 375-750 Da (%) | 20.1 | 24.5 | 24.2 | 35.9 | 20.4 | 22.9 |
| 750-1250 Da (%) | 18.2 | 15.3 | 20.6 | 24.6 | 17 | 18.8 |
| 1250-2500 Da (%) | 29.5 | 29.1 | 23.3 | 21.4 | 27.3 | 26.9 |
| >2500 Da (%) | 11.7 | 17.6 | 9.6 | 2.1 | 22.4 | 17.9 |
| DH (%) | 31.5 | 22.1 | 31.5 | 27.7 | 23.3 | 25.4 |
| FAA (mg/100 g protein (Nx6.38)) | 13825 | 4404 | 12241 | 413 | 7266 | 5936 |

The size exclusion chromatography data are not accurately accounting for free amino acids as data were acquired by measurements at 214 nm where primarily peptide bonds are expected to absorb.

Therefore, the content of free amino acids was measured by a different method (see example 10). As compared to the reference whey protein hydrolysate in sample 13, the whey protein hydrolysates according to the present invention have a higher content of free amino acids. However, the content of small peptides is higher in the reference whey protein hydrolysate (sample 13) than in the whey protein hydrolysates of the invention. For example, the content of peptides being 750 Da or less is in sample 13 more than 50%. On the contrary, the content of peptides in the whey protein hydrolysates of the present invention having a size of 750 Da or less is below 40%. Furthermore, sample 13 comprises less peptides being 2500 Da or above than the whey protein hydrolysates of the invention.

When these data are compared to the data of example 8, it becomes obvious that the low overall phenylalanine content in beta-lactoglobulin, alpha-lactalbumin and beta-casein derived peptides of samples 2, 15 and 16 is the result of these phenylalanine residues being released from the peptides into the free amino acids fraction. Hence, a method to reduce bitterness in hydrolysates may be to identify enzyme combinations that specifically concentrate phenylalanine in the free amino acids fraction of the total hydrolysate.

**Example 10: Measured free amino acid content**

The free amino acid content in the whey protein hydrolysates of the present invention was measured and compared to a reference whey protein hydrolysate (outside the invention). The result is shown in table 7 below.

Table 7: Free amino acid content (mg/100 g protein (Nx6.38))

| | Sample 1 | Sample 2 | Sample 3 | Sample 13 | Sample 15 | Sample 16 |
|---|---|---|---|---|---|---|
| Aspartic acid | 113.51 | <10 | 93.24 | <10 | 32.56 | 17.70 |
| Threonine | 898.44 | 178.05 | 850.42 | 22.72 | 282.30 | 249.70 |
| Serine | 397.09 | 57.51 | 374.84 | 17.34 | 173.36 | 117.33 |
| Glutamine | 222.56 | 31.86 | 181.56 | <10 | 194.45 | 117.21 |
| Glutamic acid | 87.62 | <10 | 101.68 | <10 | 42.29 | 29.33 |
| Proline | <10 | <10 | <10 | <10 | 16.52 | 24.61 |
| Glycine | 42.05 | 26.59 | 24.48 | <10 | 21.20 | 21.70 |
| Alanine | 663.0 | 189.76 | 445.12 | 32.68 | 343.21 | 253.33 |
| Cystine | <10 | <10 | <10 | <10 | <10 | <10 |
| Valine | 1171.37 | 480.26 | 830.50 | <10 | 730.94 | 627.88 |
| Methionine | 1055.41 | 473.23 | 1024.95 | <10 | 500.18 | 386.67 |
| Isoleucine | 1311.94 | 524.77 | 981.61 | 44.75 | 763.73 | 665.45 |

(continued)

|  | Sample 1 | Sample 2 | Sample 3 | Sample 13 | Sample 15 | Sample 16 |
|---|---|---|---|---|---|---|
| Leucine | 3736.68 | 1229.94 | 3127.56 | 152.28 | 2389.60 | 1903.03 |
| Tyrosine | 317.44 | 42.76 | 274.10 | 30.81 | 102.85 | 78.30 |
| Phenylalanine | <10 | 183.91 | <10 | <10 | 329.16 | 250.91 |
| Lysine | 1862.48 | 722.74 | 2237.32 | 81.76 | 742.65 | 723.64 |
| Histidine | 440.44 | 88.79 | 358.44 | 17.45 | 88.44 | 115.52 |
| Arginine | 957.01 | 138.22 | 715.71 | 13.47 | 384.21 | 266.67 |
| Tryptophan | 339.70 | 16.52 | 361.95 | <10 | 128.85 | 87.64 |
| Asparagine | 208.50 | 19.91 | 257.70 | <10 | 121.82 | 59.15 |
| Sum | 13825 | 4404 | 12241 | 413.26 | 7266 | 5936 |

From table 7, it is shown that the free amino acid content in the whey protein hydrolysates of the invention is from 4% to 14% by weight of the total protein content. On the contrary, the free amino acid content in the reference hydrolysate (sample 13) is about 0.4% by weight of the total protein content.

Furthermore, table 7 shows that the content of free leucine in the whey protein hydrolysates of the invention is much higher than the free leucine in sample 13. The content of free leucine in the whey protein hydrolysates of the invention is from 1 to 4% by weight of the total protein content. On the contrary, the content of free leucine in sample 13 is about 0.15% by weight of the total protein content.

More importantly, the concentration of free phenylalanine as suggested in examples 8 and 9 are higher in samples 2, 15 and 16 than in samples 1, 3 and 13. In table 8 the percentage of free amino acids based on the total amino acid content is shown.

Table 8: Free amino acid (%) of total amino acids

|  | Sample 1 | Sample 2 | Sample 3 | Sample 13 | Sample 15 | Sample 16 |
|---|---|---|---|---|---|---|
| Aspartic acid/ Asparagine | 2.00 | 0.19 | 2.47 | 0.00 | 1.48 | 0.76 |
| Threonine | 12.53 | 2.48 | 11.86 | 0.30 | 3.94 | 3.55 |
| Serine | 8.57 | 1.24 | 8.09 | 0.36 | 3.74 | 2.59 |
| Glutamine/ Glutamic acid | 1.26 | 0.18 | 1.03 | 0.00 | 1.34 | 0.81 |
| Proline | 0.00 | 0.00 | 0.00 | 0.00 | 0.27 | 0.41 |
| Glycine | 2.88 | 1.82 | 1.68 | 0.00 | 1.45 | 1.52 |
| Alanine | 12.30 | 3.52 | 8.26 | 0.59 | 6.37 | 4.72 |
| Cystine | 0.00 | 0.00 | 0.00 | 0.00 | 0.00 | 0.00 |
| Valine | 20.18 | 8.27 | 14.31 | 0.00 | 12.59 | 10.93 |
| Methionine | 46.79 | 20.98 | 45.44 | 0.00 | 22.17 | 15.96 |
| Isoleucine | 20.16 | 8.06 | 15.08 | 0.70 | 11.73 | 10.25 |
| Leucine | 35.75 | 11.77 | 29.93 | 1.55 | 22.86 | 18.07 |
| Tyrosine | 11.84 | 1.60 | 10.23 | 1.31 | 3.84 | 2.97 |
| Phenylalanine | 0.00 | 6.61 | 0.00 | 1.36 | 11.83 | 9.04 |
| Lysine | 19.80 | 7.68 | 23.79 | 0.82 | 7.90 | 7.77 |
| Histidine | 29.56 | 5.96 | 24.06 | 1.12 | 5.94 | 7.66 |
| Arginine | 47.67 | 6.89 | 35.65 | 0.75 | 19.14 | 14.59 |
| Tryptophan | 20.39 | 0.99 | 21.73 | 0.00 | 7.73 | 4.76 |

It is especially notable, that the percentage of free leucine of the total leucine content in the whey protein hydrolysates of the invention is much higher than in the reference hydrolysate (sample 13).

Importantly, table 8 shows that for the whey protein hydrolysates according to the invention in sample 2, 15 and 16, between 6 and 12% of the phenylalanine was in the form of free phenylalanine. For the whey protein hydrolysates according to the invention in sample 1 and 3, phenylalanine was not found in its free form.

**Example 11: Content of minerals in the whey protein hydrolysates of the invention**

The amount of minerals in sample 15 and 16 was measured. The result is shown in table 9 below.

Table 9: Content of minerals

| Sample | K (%) | Na (%) | Ca (%) | Citrate (%) | Ash (%) | Turbidity at 4% protein (NTU) |
|--------|-------|--------|--------|-------------|---------|-------------------------------|
| 15 | 2.13 | 1.75 | 0.07 | 0.66 | 7,5 | 31.18 |
| 16 | 2.45 | 1.6 | 0.09 | 0.65 | 7.2 | 58.68 |

Table 9 shows the mineral content and turbidity of samples 15 and 16 as an example of the mineral content of the whey protein hydrolysates. The products in table 9 all have a pH of 7.8 in a 4% protein solution at 22°C.

**Example 12: Non-degraded BSA in hydrolysates**

The amount of non-degraded bovine serum albumin (BSA) was measured in the whey protein hydrolysates of the invention (sample 1, 2, 3, 4, 15 and 16)

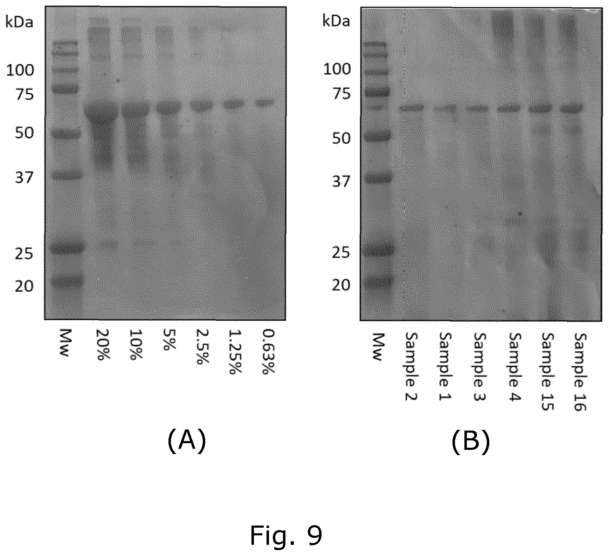

The content of BSA was estimated by using SDS-PAGE and a BSA standard from Sigma Aldrich with product code A2153 (Figure 9A). The amount of BSA loaded into the 20% well corresponded to 10 $\mu$g of pure BSA. The total amount of protein added to each well of the SDS-PAGE gel shown in Figure 9B corresponded to 50 $\mu$g of protein. Protein samples were mixed at 10 mg/ml with Laemmli sample buffer and 2-merchaptoethanol to a final concentration of 3% protein followed by incubation at 95°C for 5 minutes before loading into the gels.

The SDS-PAGE gel in Figure 9A is a titration series showing the expected intensity of BSA if it represents 20%, 10%, 5%, 2.5%, 1.25% or 0.63% of the total amount of whey protein in Figure 9B. The SDS-PAGE gel of the standard in different concentrations was compared to the SDS-PAGE gel shown in Figure 9B with different samples of whey protein hydrolysates, from left to right: a molecular weight standard, sample 2, sample 1, sample 3, sample 4, sample 15 and sample 16.

It can be concluded from figure 9A and 9B that the non-degraded BSA in the whey protein hydrolysates of the invention (Figure 9B) is in the range from 0.5 to 2% by weight as the intensity of the bands in Figure 9B corresponded to the intensity of samples at 0.63% and 1.25% in Figure 9A. The BSA was resistant to proteolysis by the enzymes used according to the invention.

**Example 13: UHT treated beverage for sports nutrition**

Example 13 shows an example of the use of the whey protein hydrolysate according to the invention in the preparation of a beverage suitable for use in sports nutrition. The beverage is intended to be used by athletes or in other sports or exercise related applications.

A powder of sample 16 was reconstituted in water and added sugars and flavours to prepare a beverage. The amounts of the ingredients are shown in table 10 below.

Table 10 shows a neutral tasting beverage with no unpleasant bitter taste that could be heat treated by direct and indirect UHT treatment as well as pasteurization without developing further turbidity.

Table 10: Sports beverage

| Ingredient | g/100 g |
|------------|---------|
| Powder of sample 16 | 4.9 |
| Sucrose | 2 |
| Sucralose | 0.006 |
| Pineapple flavour | 0.08 |

(continued)

| Ingredient | g/100 g |
|------------|---------|
| Lime flavour | 0.17 |
| Water | 92.9 |

The sports beverage shown in table 10 was subjected to 1) direct UHT, 2) indirect UHT and 3) pasteurization. Direct UHT treatment was done by injection at 143°C for 6 seconds. Indirect UHT treatment was done on a tubular heat exchanger at 143°C for 6 seconds. Pasteurization was at 90°C for 6.5 minutes. Beverages were tapped into flasks at 5°C. All solutions had a pH of about 7.7 at 22 °C. The content of 4.9 g/100g of whey protein hydrolysate of the invention corresponds to 4.0% by weight protein.

In figure 8 are pictures shown from left to right of the non-treated beverage, the direct UHT treated beverage, the indirect UHT treated beverage and the pasteurized beverage. The beverages are at room temperature. Figure 8 shows that all the samples were clear and transparent and the background behind the bottles can be seen.

The nephelometric turbidity of the 4 beverages was measured and the result is shown in table 11.

Table 11: Turbidity of beverages measured in NTU

| | Non-treated | Direct UHT | Indirect UHT | Pateurized |
|---|---|---|---|---|
| 1. sample | 58.60 | 52.22 | 73.79 | 39.93 |
| 2. sample | 59.22 | 54.41 | 71.76 | 39.88 |

Thus, from table 11, it is shown that the turbidities of the heat treated samples were all below 100 NTU. Hence, heat treatment did not affect the turbidity of the beverages which were still clear or transparent in appearance.

These beverages were not perceived as being bitter.

**Example 14: Beverage for clinical/medical use**

Example 14 is an example of the use of the whey protein hydrolysate according to the invention in the preparation of a beverage suitable for use in medical or clinical nutrition. The clinical beverage comprises a high amount of carbohydrates, besides from the whey protein hydrolysate of the invention.

A powder of sample 16 was reconstituted in water and added carbohydrates and flavourings to prepare a beverage. The amounts of the ingredients are shown in table 12 below.

Table 12 shows neutral tasting beverage for medical use with no unpleasant bitter taste.

Table 12: Medical beverage

| Ingredient | g/100 g |
|------------|---------|
| Powder of sample 16 | 4.9 |
| Carbohydrates | 33.0 |
| Fat | 5.0 |
| Sucrose | 2 |
| Sucralose | 0.006 |
| Pineapple flavour | 0.08 |
| Lime flavour | 0.17 |
| Water | 54.8 |

**Example 15: Carbonated beverage**

Example 15 shows an example of the use of the whey protein hydrolysate according to the invention in the preparation of a carbonated beverage.

A powder of sample 16 was reconstituted in water and added sugars and flavours to prepare a beverage. The amounts of the ingredients are shown in table 13 below. The beverage was carbonated by adding carbondioxide to the beverage until the beverage comprised carbon dioxide in an amount of 2.5 volumes per volume of the beverage.

Table 13: Carbonated beverage

| Ingredient | g/100 g |
|---|---|
| Powder of sample 16 | 4.9 |
| Sucrose | 2 |
| Sucralose | 0.006 |
| Pineapple flavour | 0.08 |
| Lime flavour | 0.17 |
| Water | 92.9 |
| Carbondioxide (volume per volume beverage) | 2.5 |

**Example 16: Carbonation - how the amount of carbonation influences pH**

Sample 16 was was resuspended in water to make a 8% protein solution. The solution was force carbonated at 5°C.

The mass increase and the pH value of the solution was measured over time as more $CO_2$ was introduced. When the pressure at quilibrium in the container reached about 1 bar, no more $CO_2$ could be absorbed (pressure used under force carbonation was 3 bar at 5°C).

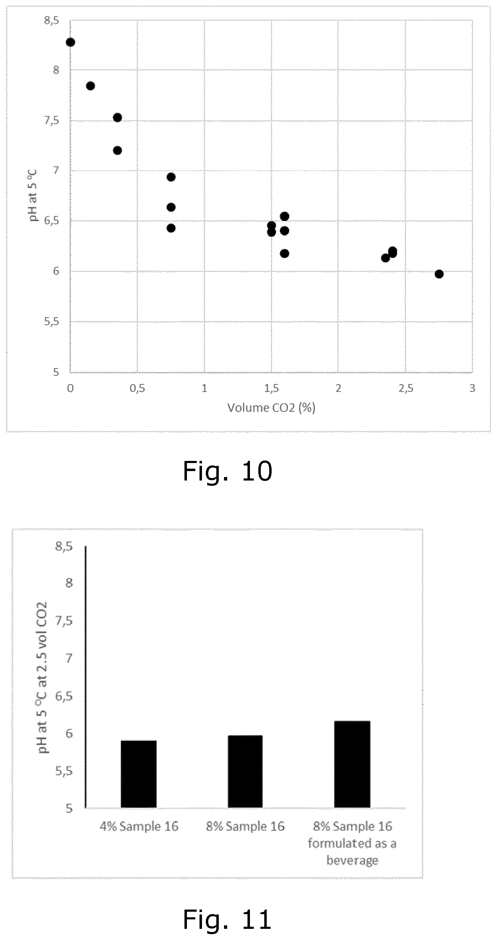

In figure 10 is the measured pH shown dependent of the amount of $CO_2$ added to the carbonated solution. Figure 10 shows that when a solution comprising the whey protein hydrolysate of sample 16 is carbonated to a $CO_2$ content of 0 to 4 volumes per volume solution, it results in a pH between 5.5 and 8.25. The pH of the non-carbonated solution has a pH value of 8.25 and the pH value decreased as the amount of carbonation increased.

Figure 10 shows that adding $CO_2$ to about 2.5 volumes (4.9 g/L) per volume solution resulted in a drop of pH from 8.25 to about 6.0 at 5°C.

Figure 10 also shows that the pH reaches a minimum of about 5.5 to 6.0. Hence, it may be concluded that addition of $CO_2$ to an amount exceeding 2.5 volumes per volume solution would not decrease the pH to less than about 5.5 to 6.0.

The effect of carbonation on the pH value of a solution or beverage was also analysed by measuring the pH of the following solutions/beverages carbonated to an amount of 2.5 volumes $CO_2$ per volume solution/beverage:

- A 4% protein solution prepared by resuspension of sample 16 in water
- A 8% protein solution prepared by resuspension of sample 16 in water
- A beverage comprising 8% of sample 16

The result is shown in figure 11.

The beverage comprising 8% sample 16 comprises the following ingredients:

| Ingredient | g/100 g |
|---|---|
| Powder of sample 16 | 9.8 |
| Sucrose | 2 |
| Sucralose | 0.006 |
| Pineapple flavour | 0.08 |
| Lime flavour | 0.17 |
| Water | 88 |
| Carbondioxide (volume per volume beverage) | 2.5 |

Figure 11 shows that the 4% and 8% solution of sample 16 and the beverage prepared from sample 16 all have a pH of about 6.0 when carbonated to a $CO_2$ content of 2.5 volumes $CO_2$ per volume solution.

**Example 17: Analysis of heat treatment of a carbonated solution**

A solution of 8% protein of sample 16 was carbonated with 2.5 volume CO2 per volume solution as disclosed in example 16.

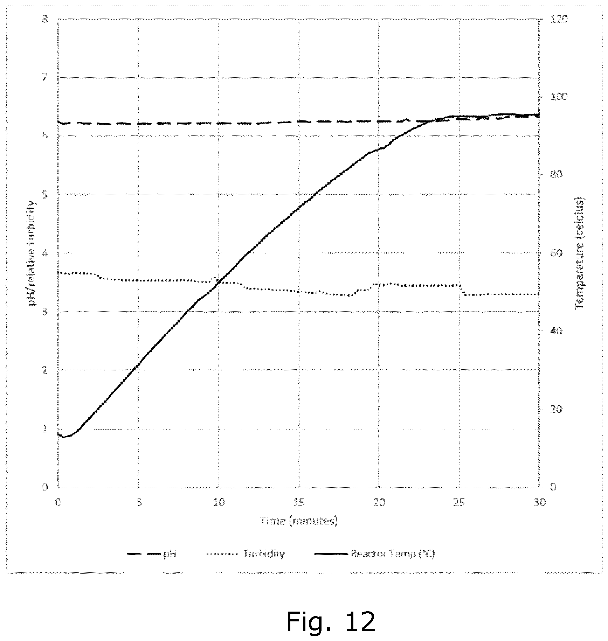

The solution was heated to a temperature of 95°C in a closed container with datalogging. The temperature, pH and turbidity was measured at different times of heating and the result is shown in figure 12.

As shown in figure 12, the carbonated product remained clear through the heating and after 5 minutes at 95°C as indicated by the measured turbidity and visual inspection. The pH remained at 6.2 through the heating. These data indicated that the carbonated product may be suitable for treatments such as pasteurization and autoclavation, essentially treatements at temperatures up to 120°C for a prolonged period, for example 20 minutes.

**Example 18: Protein bar**

Example 17 shows examples of the use of the whey protein hydrolysate according to the invention in the preparation of a protein bar.

In table 14 is an example of a protein bar having a content of 5 g/100g of the whey protein hydrolysate (powder) of the present invention.

Table 14: Protein bar

|  | g/100 g |
|---|---|
| Hydrolysate | 5 |
| Milk protein | 37 |
| Glycerol | 4,9 |
| Sugars | 39,5 |
| Lipids | 6 |
| Flavours | 1,75 |

In table 15 is another example of a protein bar shown, but this example has a content of the whey protein hydrolysate (powder) in an amount of of 13 g/100 g.

Table 15: Protein bar

|  | g/100 g |
|---|---|
| Hydrolysate | 13 |
| Milk protein | 24 |
| Glycerol | 4,9 |
| Sugars | 39,5 |
| Lipids | 6 |
| Flavours | 1,75 |

**Example 19: Analysis of antioxidative activity by use of DPPH assay**

The antioxidative effect of the whey protein hydrolysate of the invention was analysed by using the DPPH assay that is a radical scavenging assay. The assay measures antioxidative effect by using DPPH (2,2-diphenyl-1-picrylhydrazyl-hydrate), since the color of DPPH changes from purple to yellow upon reaction with antioxidative peptides.

DPPH was dissolved in methanol to a concentration of 0.2 mM. The whey protein hydrolysate of the invention (sample 16) was dispersed in MilliQ water to different contentrations of protein (0, 0.8, 1.5, 2.5, 3.0, 4.0, 5.0 and 6.0 % protein) and mixed with DPPH in equal volumes (ratio 1:1). The mixtures were incubated for 2 hours at 22°C to allow yellow color to develop as a result of the antioxidative activity. The absorbance at 525 nm was measured and the scavenging percentage calculated as $100 \times (A_0 - A_S)/A_0$, where $A_0$ is the absorption in the absence of sample, and As is the absorbance in the presence of sample.

Table 16 shows the scavenging percentage for various concentrations of the whey protein hydrolysate of the invention (sample 16).

Table 16:

| Sample 16 (% protein) | Scavenging percentage (%) |
|---|---|
| 0 | 0 |
| 0.8 | $48 \pm 2$ |
| 1.5 | $57 \pm 3$ |
| 2.5 | $67 \pm 1$ |
| 3 | $72 \pm 1$ |
| 4 | $75 \pm 2$ |
| 5 | $77 \pm 2$ |
| 6 | $78 \pm 0$ |

From table 16, it is shown that the whey protein hydrolysate of sample 16 has an antioxidative activity when included in the DPPH assay at concentrations from 0.8 to 6% protein. As shown in table 16, the antioxidative activity increased as the concentration of protein increased.

**Claims**

1. A method of preparing a whey protein hydrolysate comprising:

   a) providing a whey protein solution comprising whey protein in an amount of at least 50% by weight based on the total solid content;
   b) subjecting the whey protein solution to enzymatic hydrolysis, wherein the enzymatic hydrolysis is performed with the use of either one of the following enzyme combinations;

   i) comprising at least a serine endopeptidase from *Bacillus,* at least a serine endopeptidase from *Aspergillus,* and at least a trypsin-like protease
   ii) comprising at least a serine endopeptidase from *Bacillus,* at least a serine endopeptidase from *Aspergillus,* and at least a leucyl aminopeptidase from *Aspergillus*
   iii) comprising at least a bacillolysin from *Bacillus amyloliquefaciens,* at least bromelain, and at least a leucyl aminopeptidase from *Aspergillus*

   c) stopping the enzymatic hydrolysis by inactivating the enzymes when the degree of hydrolysis (DH) is 20% or more to obtain a whey protein hydrolysate; wherein the method does not comprise any step of ultrafiltration of the whey protein hydrolysate obtained in step c).

2. The method according to claim 1, wherein the method furthermore comprises a step d) of concentrating and/or drying the whey protein hydrolysate obtained in step c).

3. The method according to claims 1 or 2, wherein the whey protein solution of step a) comprises lipids in an amount of at most 10% by weight based on the total solid content.

4. The method according to claim 1, wherein the enzymatic hydrolysis in step b) is performed at a temperature in the range of 40°C to 75°C.

5. The method according to any of the claims 1 to 4, wherein the whey protein solution comprises protein in an amount of 2% by weight or more of the whey protein solution.

6. The method according to any of the claims 1 to 5, wherein the enzymatic hydrolysis in step c) is stopped by inactivating the enzymes when the degree of hydrolysis (DH) is in the range of from 20% to 35%.

7. The method according to any of the claims 1 to 6, wherein the enzymatic hydrolysis in step b) is performed with the use of either one of the following enzyme combinations:

    i. comprising at least a serine endopeptidase from a *Bacillus species,* at least a serine endopeptidase from *Aspergillus oryzae,* and at least a trypsin-like protease of microbial origin
    ii. comprising at least a subtilisin from *Bacillus licheniformis,* at least a serine endopeptidase from *Aspergillus oryzae,* and at least a leucyl aminopeptidase from *Aspergillus oryzae*
    iii. comprising at least a bacillolysin from *Bacillus amyloliquefaciens,* at least bromelain from *Ananas comosus,* and at least a leucyl aminopeptidase from *Aspergillus oryzae*

8. A whey protein hydrolysate comprising:

    - free amino acids and peptides, and
    - having a degree of hydrolysis of at least 20%
    - peptides having a molecular weight of 2500 Da or more in an amount of 8 to 25% by weight of the total amount of peptides, and
    - having free amino acids in an amount of 15% by weight or less of the total amino acid content in the hydrolysate, and

wherein the whey protein hydrolysate in a 4% w/w protein solution has a bitterness score corresponding to a solution of 0.08% w/v or less of caffeine.

9. A whey protein hydrolysate according to claim 8, wherein the degree of hydrolysis is from 20% to 35%.

10. The whey protein hydrolysate according to any of claims 8 or 9, wherein the whey protein hydrolysate has a nephelometric turbidity (NTU) of 100 or below in a 4% (w/w) protein solution.

11. The whey protein hydrolysate according to any of the claims 8 to 10, wherein the whey protein hydrolysate comprises free amino acids in an amount of 2-15% by weight of the total protein content in the hydrolysate.

12. The whey protein hydrolysate according to any of the claims 8 to 11, wherein the whey protein hydrolysate has an antioxidative activity.

13. The whey protein hydrolysate according to claim 8 to 12, wherein the antioxidative activity of the whey protein hydrolysate is measured as having a scavenging percentage of 54 to 60 in a 1.5% by weight protein solution.

14. Use of the whey protein hydrolysate according to any of the claims 8 to 13 in a food product.

15. Use according to claim 14, wherein the whey protein hydrolysate is used in the food product in an amount corresponding to the food product comprises from 2 to 25% by weight hydrolysed protein.

16. Use according to any of claims 14 or 15, wherein the food product is selected from the group consisting of a dairy product, a beverage, a shake, a gel, a shot and a food bar.

17. Use of the whey protein hydrolysate according to any of claims 8 to 13 as a food ingredient.

18. Use of the whey protein hydrolysate according to any of claims 8 to 13 as a food ingredient in the preparation of UHT stable beverage having a pH of 6.5-8.0.

19. Use of the whey protein hydrolysate according to any of the claims 8 to 13 as a food ingredient in the preparation of beverages for use as sports nutrition.

20. Use of the whey protein hydrolysate according to any of the claims 8 to 13 as a food ingredient in the preparation of a clinical beverage.

21. Use of the whey protein hydrolysate according to any of the claims 8 to 13 as an ingredient in the preparation of a carbonated beverage.

**22.** Use of the whey protein hydrolysate according to any of the claims 8 to 13 as an antioxidant.

**23.** A carbonated beverage comprising whey protein hydrolysate according to any of the claims 8 to 13.

**Patentansprüche**

**1.** Verfahren zum Herstellen eines Molkeproteinhydrolysats, umfassend:

a) Bereitstellen einer Molkeproteinlösung, die Molkeprotein in einer Menge von mindestens 50 Gew.-%, bezogen auf den Gesamtfeststoffgehalt, umfasst;
b) Unterziehen der Molkeproteinlösung einer enzymatischen Hydrolyse, wobei die enzymatische Hydrolyse unter Verwendung einer der folgenden Enzymkombinationen durchgeführt wird;

i) umfassend mindestens eine Serin-Endopeptidase aus *Bacillus,* mindestens eine Serin-Endopeptidase aus *Aspergillus* und mindestens eine Trypsin-ähnliche Protease
ii) umfassend mindestens eine Serin-Endopeptidase aus *Bacillus,* mindestens eine Serin-Endopeptidase aus *Aspergillus* und mindestens eine Leucin-Aminopeptidase aus *Aspergillus*
iii) umfassend mindestens ein Bacillolysin aus *Bacillus amyloliquefaciens,* mindestens Bromelain und mindestens eine Leucin-Aminopeptidase aus *Aspergillus*

c) Anhalten der enzymatischen Hydrolyse durch Inaktivieren der Enzyme, wenn der Hydrolysegrad (DH) 20 % oder mehr beträgt, um ein Molkeproteinhydrolysat zu erhalten;

wobei das Verfahren keinen Schritt von Ultrafiltration des in Schritt c) erhaltenen Molkeproteinhydrolysats umfasst.

**2.** Verfahren nach Anspruch 1, wobei das Verfahren ferner einen Schritt d) eines Konzentrierens und/oder Trocknens des in Schritt c) erhaltenen Molkeproteinhydrolysats umfasst.

**3.** Verfahren nach Anspruch 1 oder 2, wobei die Molkeproteinlösung aus Schritt a) Lipide in einer Menge von höchstens 10 Gew.-%, bezogen auf den Gesamtfeststoffgehalt, umfasst.

**4.** Verfahren nach Anspruch 1, wobei die enzymatische Hydrolyse in Schritt b) bei einer Temperatur in dem Bereich von 40 °C bis 75 °C durchgeführt wird.

**5.** Verfahren nach einem der Ansprüche 1 bis 4, wobei die Molkeproteinlösung Protein in einer Menge von 2 Gew.-% oder mehr der Molkeproteinlösung umfasst.

**6.** Verfahren nach einem der Ansprüche 1 bis 5, wobei die enzymatische Hydrolyse in Schritt c) durch Inaktivieren der Enzyme angehalten wird, wenn der Hydrolysegrad (DH) in dem Bereich von 20 % bis 35 % liegt.

**7.** Verfahren nach einem der Ansprüche 1 bis 6, wobei die enzymatische Hydrolyse in Schritt b) unter Verwendung einer der
folgende Enzymkombinationen durchgeführt wird:

i. umfassend mindestens eine Serin-Endopeptidase aus einer *Bacillus-Spezies,* mindestens eine Serin-Endopeptidase aus *Aspergillus oryzae* und mindestens eine Trypsin-ähnliche Protease mikrobiellen Ursprungs
ii. umfassend mindestens ein Subtilisin aus *Bacillus licheniformis,* mindestens eine Serin-Endopeptidase aus *Aspergillus oryzae* und mindestens eine Leucin-Aminopeptidase aus *Aspergillus oryzae*
iii. umfassend mindestens ein Bacillolysin aus *Bacillus amyloliquefaciens,* mindestens Bromelain aus *Ananas comosus* und mindestens eine Leucin-Aminopeptidase aus *Aspergillus oryzae*

**8.** Molkeproteinhydrolysat, umfassend:

- freie Aminosäuren und Peptide und
- aufweisend einen Hydrolysegrad von mindestens 20 %
- Peptide, die ein Molekulargewicht von 2500 Da oder mehr in einer Menge von 8 bis 25 Gew.-% der Gesamtmenge der Peptide aufweisen, und

- aufweisend freie Aminosäuren in einer Menge von 15 Gew.-% oder weniger des gesamten Aminosäuregehalts in dem Hydrolysat und

wobei das Molkeproteinhydrolysat in einer Proteinlösung von 4 % w/w einen Bitterkeitswert aufweist, der einer Lösung von 0,08 % w/v oder weniger Koffein entspricht.

9. Molkeproteinhydrolysat nach Anspruch 8, wobei der Hydrolysegrad 20 % bis 35 % beträgt.

10. Molkeproteinhydrolysat nach einem der Ansprüche 8 oder 9, wobei das Molkeproteinhydrolysat eine nephelometrische Trübung (NTU) von 100 oder weniger in einer Proteinlösung von 4% (w/w) aufweist.

11. Molkeproteinhydrolysat nach einem der Ansprüche 8 bis 10, wobei das Molkeproteinhydrolysat freie Aminosäuren in einer Menge von 2 bis 15 Gew.-% des Gesamtproteingehalts in dem Hydrolysat umfasst.

12. Molkeproteinhydrolysat nach einem der Ansprüche 8 bis 11, wobei das Molkeproteinhydrolysat eine antioxidative Aktivität aufweist.

13. Molkeproteinhydrolysat nach Anspruch 8 bis 12, wobei die antioxidative Aktivität des Molkeproteinhydrolysats so gemessen wird, dass sie einen Scavenger-Prozentsatz von 54 bis 60 in einer Proteinlösung von 1,5 Gew.-% aufweist.

14. Verwendung des Molkeproteinhydrolysats nach einem der Ansprüche 8 bis 13 in einem Lebensmittelprodukt.

15. Verwendung nach Anspruch 14, wobei das Molkeproteinhydrolysat, das in dem Lebensmittelprodukt in einer Menge entsprechend dem Lebensmittelprodukt verwendet wird, 2 bis 25 Gew.-% hydrolysiertes Protein umfasst.

16. Verwendung nach einem der Ansprüche 14 oder 15, wobei das Lebensmittelprodukt aus der Gruppe ausgewählt ist, die aus einem Molkereiprodukt, einem Getränk, einem Shake, einem Gel, einem Shot und einem Lebensmittelriegel besteht.

17. Verwendung des Molkeproteinhydrolysats nach einem der Ansprüche 8 bis 13 als Lebensmittelbestandteil.

18. Verwendung des Molkeproteinhydrolysats nach einem der Ansprüche 8 bis 13 als Lebensmittelbestandteil bei der Herstellung eines UHT-stabilen Getränks mit einem pH-Wert von 6,5-8,0.

19. Verwendung des Molkeproteinhydrolysats nach einem der Ansprüche 8 bis 13 als Lebensmittelbestandteil bei der Herstellung von Getränken zur Verwendung als Sportnahrung.

20. Verwendung des Molkeproteinhydrolysats nach einem der Ansprüche 8 bis 13 als Lebensmittelbestandteil bei der Herstellung eines klinischen Getränks.

21. Verwendung des Molkeproteinhydrolysats nach einem der Ansprüche 8 bis 13 als Bestandteil bei der Herstellung eines kohlensäurehaltigen Getränks.

22. Verwendung des Molkeproteinhydrolysats nach einem der Ansprüche 8 bis 13 als Antioxidans.

23. Kohlensäurehaltiges Getränk, umfassend Molkeproteinhydrolysat nach einem der Ansprüche 8 bis 13.


**Revendications**

1. Procédé de préparation d'un hydrolysat de protéines de lactosérum comprenant :

   a) la fourniture d'une solution de protéines de lactosérum comprenant des protéines de lactosérum en une quantité supérieure ou égale à 50 % en poids sur la base de la teneur totale en solides ;
   b) la soumission de la solution de protéines de lactosérum à une hydrolyse enzymatique, ladite hydrolyse enzymatique étant réalisée à l'aide de l'une ou l'autre des combinaisons d'enzymes suivantes ;

      i) comprenant au moins une sérine endopeptidase de *Bacillus,* au moins une sérine endopeptidase *d'As-*

*pergillus,* et au moins une protéase de type trypsine

ii) comprenant au moins une sérine endopeptidase de *Bacillus,* au moins une sérine endopeptidase *d'Aspergillus,* et au moins une leucyl-aminopeptidase *d'Aspergillus*

iii) comprenant au moins une bacillolysine de *Bacillus amyloliquefaciens,* au moins de la bromélaïne, et au moins une leucyl aminopeptidase *d'Aspergillus*

c) l'arrêt de l'hydrolyse enzymatique en inactivant les enzymes lorsque le degré d'hydrolyse (DH) est supérieur ou égal à 20 % pour obtenir un hydrolysat de protéines de lactosérum ;

ledit procédé ne comprenant aucune étape d'ultrafiltration de l'hydrolysat de protéines de lactosérum obtenu à l'étape c).

2. Procédé selon la revendication 1, ledit procédé comprenant en outre une étape d) de concentration et/ou de séchage de l'hydrolysat de protéines de lactosérum obtenu à l'étape c).

3. Procédé selon les revendications 1 ou 2, ladite solution de protéines de lactosérum de l'étape a) comprenant des lipides en une quantité inférieure ou égale à 10 % en poids sur la base de la teneur totale en solides.

4. Procédé selon la revendication 1, ladite hydrolyse enzymatique à l'étape b) étant réalisée à une température comprise dans la plage de 40°C à 75°C.

5. Procédé selon l'une quelconque des revendications 1 à 4, ladite solution de protéines de lactosérum comprenant des protéines en une quantité supérieure ou égale à 2 % en poids de la solution de protéines de lactosérum.

6. Procédé selon l'une quelconque des revendications 1 à 5, ladite hydrolyse enzymatique à l'étape c) étant arrêtée en inactivant les enzymes lorsque le degré d'hydrolyse (DH) est compris dans la plage de 20 % à 35 %.

7. Procédé selon l'une quelconque des revendications 1 à 6, ladite hydrolyse enzymatique à l'étape b) étant réalisée à l'aide de l'une ou l'autre des combinaisons d'enzymes suivantes :

i. comprenant au moins une sérine endopeptidase d'une *espèce de Bacillus,* au moins une sérine endopeptidase *d'Aspergillus oryzae,* et au moins une protéase de type trypsine d'origine microbienne

ii. comprenant au moins une subtilisine de *Bacillus licheniformis,* au moins une sérine endopeptidase *d'Aspergillus oryzae,* et au moins une leucyl aminopeptidase *d'Aspergillus oryzae*

iii. comprenant au moins une bacillolysine de *Bacillus amyloliquefaciens,* au moins de la bromélaïne *d'Ananas comosus,* et au moins une leucyl aminopeptidase *d'Aspergillus oryzae.*

8. Hydrolysat de protéines de lactosérum comprenant :

- des acides aminés et des peptides libres, et
- possédant un degré d'hydrolyse supérieur ou égal à 20 %
- des peptides possédant un poids moléculaire supérieur ou égal à 2500 Da en une quantité de 8 à 25 % en poids de la quantité totale de peptides, et
- possédant des acides aminés libres en une quantité inférieure ou égale à 15 % en poids de la teneur totale en acides aminés dans l'hydrolysat, et

ledit hydrolysat de protéines de lactosérum dans une solution de protéines à 4 % p/p possédant un score d'amertume correspondant à une solution de 0,08 % p/v ou moins de caféine.

9. Hydrolysat de protéines de lactosérum selon la revendication 8, ledit degré d'hydrolyse allant de 20 % à 35 %.

10. Hydrolysat de protéines de lactosérum selon l'une quelconque des revendications 8 ou 9, ledit hydrolysat de protéines de lactosérum possédant une turbidité néphélométrique (NTU) inférieure ou égale à 100 dans une solution de protéines à 4 % (p/p).

11. Hydrolysat de protéines de lactosérum selon l'une quelconque des revendications 8 à 10, ledit hydrolysat de protéines de lactosérum comprenant des acides aminés libres en une quantité de 2-15 % en poids de la teneur totale en protéines dans l'hydrolysat.

**12.** Hydrolysat de protéines de lactosérum selon l'une quelconque des revendications 8 à 11, ledit hydrolysat de protéines de lactosérum possédant une activité antioxydante.

**13.** Hydrolysat de protéines de lactosérum selon les revendications 8 à 12, ladite activité antioxydante de l'hydrolysat de protéines de lactosérum étant mesurée comme possédant un pourcentage de piégeage de 54 à 60 dans une solution de protéines à 1,5 % en poids.

**14.** Utilisation de l'hydrolysat de protéines de lactosérum selon l'une quelconque des revendications 8 à 13 dans un produit alimentaire.

**15.** Utilisation selon la revendication 14, ledit hydrolysat de protéine de lactosérum étant utilisé dans le produit alimentaire en une quantité correspondant au produit alimentaire comprenant de 2 à 25 % en poids de protéine hydrolysée.

**16.** Utilisation selon l'une quelconque des revendications 14 ou 15, ledit produit alimentaire étant choisi dans le groupe constitué par un produit laitier, une boisson, une boisson frappée, un gel, un shot et une barre alimentaire.

**17.** Utilisation de l'hydrolysat de protéines de lactosérum selon l'une quelconque des revendications 8 à 13 en tant qu'ingrédient alimentaire.

**18.** Utilisation de l'hydrolysat de protéines de lactosérum selon l'une quelconque des revendications 8 à 13 en tant qu'ingrédient alimentaire dans la préparation d'une boisson stable UHT possédant un pH de 6,5-8,0.

**19.** Utilisation de l'hydrolysat de protéines de lactosérum selon l'une quelconque des revendications 8 à 13 en tant qu'ingrédient alimentaire dans la préparation de boissons destinées à être utilisées en tant que nutrition sportive.

**20.** Utilisation de l'hydrolysat de protéines de lactosérum selon l'une quelconque des revendications 8 à 13 en tant qu'ingrédient alimentaire dans la préparation d'une boisson clinique.

**21.** Utilisation de l'hydrolysat de protéines de lactosérum selon l'une quelconque des revendications 8 à 13 en tant qu'ingrédient dans la préparation d'une boisson gazeuse.

**22.** Utilisation de l'hydrolysat de protéines de lactosérum selon l'une quelconque des revendications 8 à 13 en tant qu'antioxydant.

**23.** Boisson gazeuse comprenant un hydrolysat de protéines de lactosérum selon l'une quelconque des revendications 8 à 13.

(A)

(B)

(C)

Fig. 1

(A)

(B)

(C)

Fig. 2

Fig. 3

Fig. 4

Fig. 5

Fig. 6

Fig. 7

Fig. 8

(A)

(B)

Fig. 9

Fig. 10

Fig. 11

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0219837 A1
- WO 2016156077 A
- WO 1993024020 A1

### Non-patent literature cited in the description

- **ADLER-NISSEN, J.** Determination of the degree of hydrolysis of food protein hydrolysates by trinitrobenzenesulfonic acid. *J. Agric. Food Chem.,* 1979, vol. 27, 1256-1262
- **NIELSEN, P. M., ; PETERSEN, D. ; DAMBMANN, C.** Improved method for determining food protein degree of hydrolysis. *J. Food Sci.,* 2001, vol. 66, 642-646
- **ADLER-NISSEN J.** *Agricultural and Food Chemistry,* 1979, vol. 27 (6), 1256
- **R. SCHUSTER.** Determination of Amino Acids in Biological, Pharmaceutical, Plant and Food Samples by Automated Precolumn Derivatization and HPLC. *Journal of Chromatography,* 1988, vol. 431, 271-284
- Rapid, Accurate, Sensitive, and Reproducible HPLC Analysis of Amino Acids. **HENDERSON, J.W. ; RICKER, R.D. ; BIDLINGMEYER, B.A. ; WOODWARD, C.,.** Amino Acid Analysis Using Zorbax Eclipse-AAA columns and the Agilent 1100 HPLC. Agilent Publication, 2000
- **LIU. X. ; JIANG. D. ; PETERSON. D.G. ; J. AGRIC. FOOD CHEM.** *Identification of Bitter Peptides in whey protein hydrolysate.,* 2014, vol. 62, 5719-5725